# EUROPEAN PATENT APPLICATION

(11) **EP 2 535 057 A2**
(43) Date of publication of application: **19.12.2012**
(21) Application number: 11715008.6
(22) Date of filing: 07.02.2011
(51) Int. Cl.: A61K 38/17, A61K 38/21

(54) **COMPOSITIONS FOR THE TREATMENT OF INFECTIOUS AND TUMOURAL DISEASES**

(30) Priority: 09.02.2010 ES 201030170
(71) Applicant: Proyecto de Biomedicina Cima, S.L., 31008 Pamplona - Navarra (ES); Digna Biotech, S.L., 31008 Pamplona Navarra (ES)
(72) Inventor: ALDABE ARREGUI, Rafael, E-31008 Pamplona - Navarra (ES); GONZÁLEZ DE LA TAJADA, Iranzu, E-31008 Pamplona - Navarra (ES); LARREA LEOZ, María Esther, E-31008 Pamplona - Navarra (ES); PRIETO VALTUEÑA, Jesús María, E-31008 Pamplona - Navarra (ES)
(74) Representative: ABG Patentes, S.L.
(86) International application number: PCT/ES2011/070077
(87) International publication number: WO 2011/098644

(57) **Abstract**

The present invention relates to the use of compositions comprising a type III interferon and oncostatin M and to the use of said compositions for the treatment and prevention of infectious and neoplastic diseases.

## Description

### TECHNICAL FIELD OF THE INVENTION

This invention relates to therapies designed for the treatment and prevention of infectious and/or neoplastic diseases and, more specifically, to therapies based on the use of interferons and combinations thereof with agents capable of stimulating the activity of said interferons.

### BACKGROUND OF THE INVENTION

Interferon (IFN) was the first cytokine identified, in 1957, by Isaacs and Lindenmann from viral interference studies (Proc. R. Soc. Lond. B. Biol. Sci., 1957, 147:258-267). It was also the first cytokine that was purified, cloned, completely sequenced and produced in a recombinant manner. The identification of several isoforms has led to the classification of interferons on the basis of their amino acid sequence and on the basis of their capacity to specifically bind to different receptors. In mammals, interferons have been classified into three families, known as type I, type II and type III interferons.

Type I interferons constitute a multigenic family with cytokine activity, and were originally discovered thanks to their inhibitory activity on the *in vitro* viral infection of cell lines (Pestka, S., Krause, C.D., and Walter, M.R. 2004. Immunol Rev. Vol. 202:8-32). On the basis of the homology of their sequences, type I interferons are grouped into at least 8 subclasses of interferons, including IFN-α, IFN-β, IFN-ε, IFN-κ, IFN-ω, IFN-τ, IFN-δ and IFN-ζ (limitin). IFN-α and IFN-β share a single dimeric receptor that is expressed on the surface of most nucleated cells. Both the human and the mouse genome contain a single gene that encodes IFN-β, whereas they contain 12 or 13 functional genes that encode IFN-α. The function of these cytokines is of great significance in the immune response against numerous types of viral infections, since they set in motion mechanisms that promote the death of the infected cells by apoptosis and the inhibition of viral replication, whilst favouring antigenic presentation. Recently, it has been experimentally documented that they also exert their functions by directly activating the activities of T, B and NK lymphocytes, as well as of dendritic cells in the immune response (Le Bon A. et al., 2003. Nat. Immunol. Vol. 4:1009-15; Le Bon A. et al., 2006. J. Immunol. Vol. 176:4682-4689; Le Bon A. et al., 2006. J Immunol. 176:2074-8).

Type II interferon consists of a single interferon, IFN-γ.

Recently, a new family of IFNs has been identified, formed by IFN-λ1, IFN- λ2 and IFN-λ3, which are collectively called type III interferons. The structure of these interferons is similar to that of the IL-10 family; consequently, these three molecules were independently described as IL-29 (IFN-λ1), IL-28A (IFN-λ2) and IL-28B (IFN-λ3). However, structurally and functionally, IFN-λs are more related to type I IFNs than to cytokines from the IL-10 family, since they are capable of activating ISRE and inducing antiviral activity.

Type I IFNs are capable of generating an antiviral response thanks to their capacity to induce the expression of class I histocompatibility antigens and the induction of antiviral protection, as well as the induction of a number of genes, generically called ISGs (interferon-stimulated genes), in different cell lines, many of which encode proteins with antiviral activity, such as the dsRNA-activated threonine/serine protein kinase, the 2',5'-OAS protein and the MxA protein.

Recently, it has been shown that IFN-α is capable of increasing the antigenicity of infected or tumoural epithelial cells by means of an increase in antigen processing and presentation in these cells, making these cells more visible and reactive toward immune responses in a suicide-type mechanism that leads to a selective destruction of the same cells. This activity of IFN-α is increased by the co-administration of oncostatin M. Thus, Larrea et al. (J. Virol. 2009, 83:3298-3311) have shown that OSM is capable of increasing the antiviral effect of IFN-α in Huh7 hepatoma cells infected with the hepatitis A or the hepatitis C virus.

On the other hand, W02006/134195 discloses, for the first time, the use of an interferon-alpha in a pharmaceutical composition designed for combined administration with an IL-6 family cytokine, in particular cardiotrophin 1 (CT-1) or oncostatin M (OSM), and that said composition provides a strong synergistic antiviral effect. The combined administration of both cytokines is useful for the treatment of viral diseases, particularly against the hepatitis C virus (HCV).

Similarly, Ikeda et al. [Ikeda et al., FEBS Lett. 2009; 583:1434-1438] have disclosed that oncostatin M in combination with interferon-alpha synergically inhibits the replication of HCV RNA.

Although all these combinations allow for the administration of lower doses of IFN-α, their use is usually accompanied by a number of adverse effects as a consequence of the administration of interferon. Thus, there is a need in the state of the art for compositions and methods that make it possible to increase the antiviral and antineoplastic capacity of interferons and which do not present the disadvantages associated with the compositions known thus far.

### SUMMARY OF THE INVENTION

A first aspect of the invention relates to a composition or kit comprising, together or separately:
(i) a first component selected from the group of
   (a) an OSMR agonist and
   (b) a polynucleotide that encodes an OSMR agonist and
(ii) a second component selected from the group of
   (a) at least one type III interferon or a functionally equivalent variant thereof, and
   (b) at least one polynucleotide that encodes a type III interferon or a functionally equivalent variant thereof.

A second aspect of the invention relates to a pharmaceutical composition comprising a composition or kit of the invention and at least one pharmaceutically acceptable carrier.

A third aspect of the invention relates to a composition, a kit or a pharmaceutical composition according to the invention for use in medicine.

A fourth aspect of the invention relates to the use of a composition, a kit or a pharmaceutical composition according to the invention for the manufacture of a medicament for the treatment of an infectious disease and/or a neoplastic disease.

### DESCRIPTION OF THE FIGURES

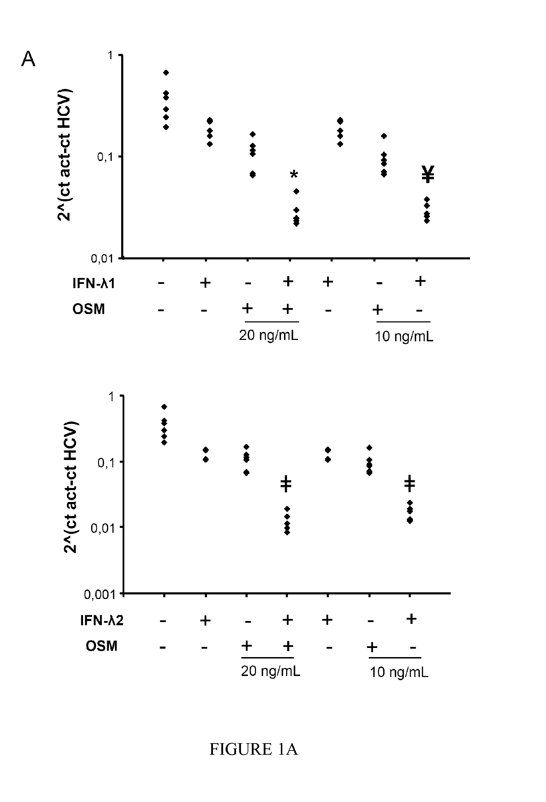

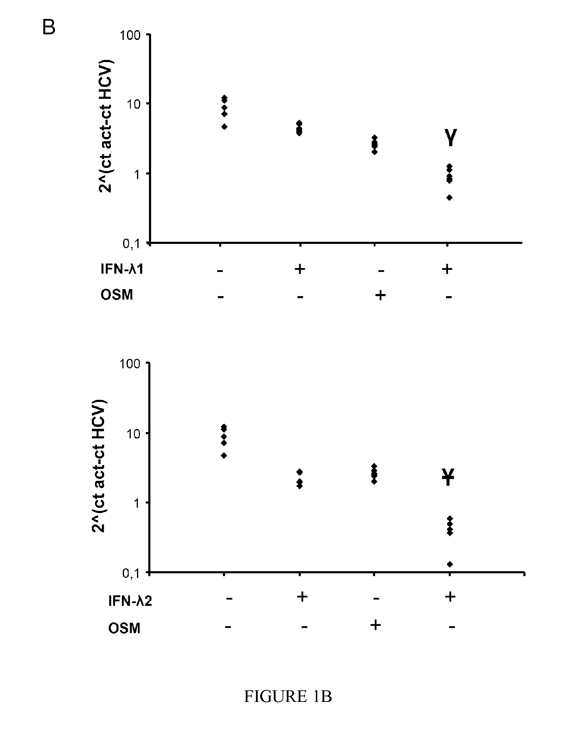

Figure 1. Quantitative analysis by means of RT-PCR of HCV messenger RNA present in two different clones of Huh7 cells that contain the complete HCV genomic replicon, following 3 days of treatment with one of the following substances to be assayed: Control, wherein the compounds were not added; IFN-λ1 (50 IU/ml); OSM (10 ng/ml); combination of IFN-λ1 (50 IU/ml) with OSM (10 or 20 ng/ml); IFN-λ2 (50 IU/ml); and combination of IFN-λ2 (50 IU/ml) with OSM (10 or 20 ng/ml) (Figs. 1A and 1B). The legend "+" next to a compound (OSM, IFN-λ1 or IFN-λ2) indicates that the substance to be assayed comprises said compound. As may be observed in the figure, combined treatment with an IFN-λ (1 or 2) plus OSM produces a greater inhibition of the viral replication of HCV than that induced by the cytokines individually administered. * P ≤0.012 vs Control, IFN-λ1 and OSM 20 ng/ml; ¥ P≤0.004 vs. Control, IFN-λ1 and OSM 10 ng/ml; ≠ P≤0.008 vs. Control, IFN-λ2 and OSM 10 ng/ml and OSM 20 ng/ml; γ P≤0.004 vs. Control, IFN-λ1 and OSM 10 ng/ml; 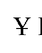 P≤0.008 vs. Control, IFN-λ2 and OSM 10 ng/ml.

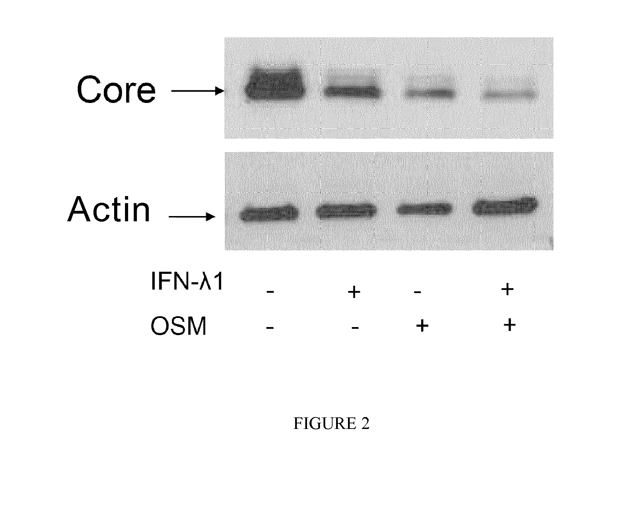

Figure 2. Western-Blot analysis of the viral Core structural protein in Huh7 cells that express the full-length HCV replicon. The cells were treated for three days with one of the following substances to be assayed: Control, wherein the compounds were not added; IFN-λ1 (50 IU/ml); OSM (10 ng/ml); and combination of IFN-λ1 (50 IU/ml) with OSM (10 ng/ml). The legend "+" next to a compound (OSM or IFN-λ1) indicates that the substance to be assayed comprises said compound. The figure shows that the detection of Core is significantly lower in the combined treatment using IFN-λ1 with OSM. The image is representative of several experiments performed on different genomic replicons.

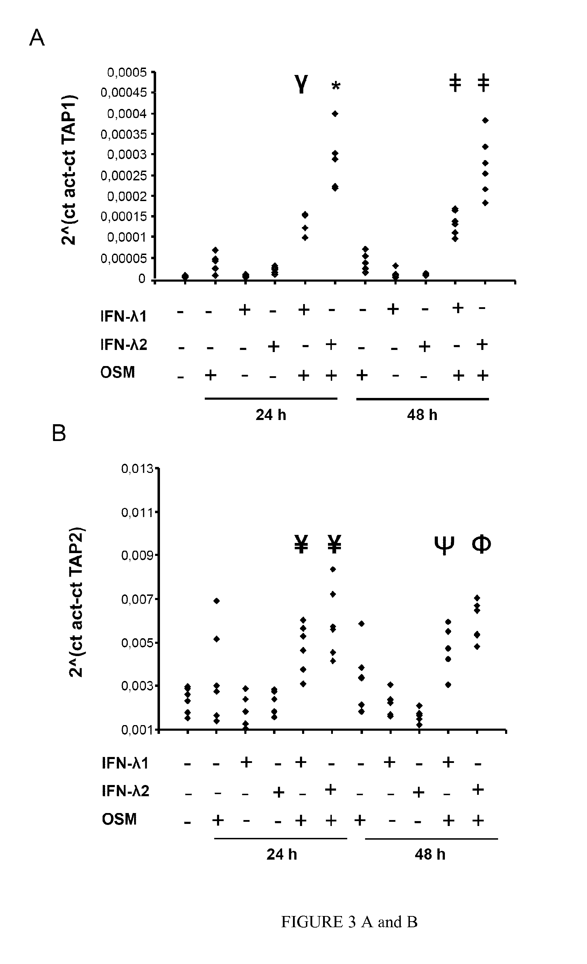

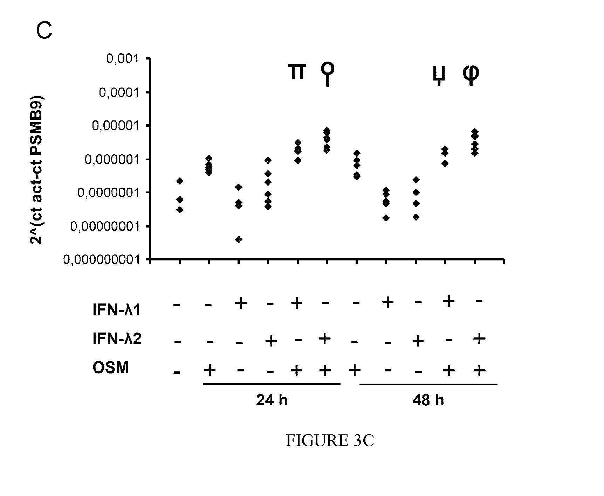

Figure 3. Quantitative analysis by means of RT-PCR of the mRNA of genes involved in immunomodulation. OSM presents synergy with IFN-λ1 and IFN-λ2 in the induction of genes involved in antigen processing and presentation, in Huh7 cells that carry the HCV genomic replicon. The figure shows the determination by means of quantitative RT-PCR of the levels of transporter mRNAs associated with antigen processing, TAP1 (A) and TAP2 (B), and immunoproteasome subunit PSMB9 (C) in cells treated for 24 and 48 hours (h) with one of the following substances to be assayed: Control, wherein no compounds were added; OSM (10 ng/ml); IFN-λ1 (50 IU/ml); IFN-λ2 (50 IU/ml); combination of IFN-λ1 (50 IU/ml) with OSM (10 ng/ml); and combination of IFN-λ2 (50 IU/ml) with OSM (10 ng/ml). The legend "+" next to a compound (OSM, IFN-λ1 or IFN-λ2) indicates that the substance to be assayed comprises said compound. γ P≤0.010 vs. Control, IFN-λ1 and OSM 24 h; * P ≤0.016 vs Control, IFN-λ2 and OSM 24 h; ‡P≤0.004 vs. Control, IFN-λ1, IFN-λ2 and OSM 48 h; ¥ P≤0.002 vs. IFN-λ1, IFN-λ2, 24 h; Ψ P ≤0.030 vs. Control, IFN-λ1 and OSM 48 h; Φ P≤0.004 vs. Control, IFN-λ2 and OSM 48 h.

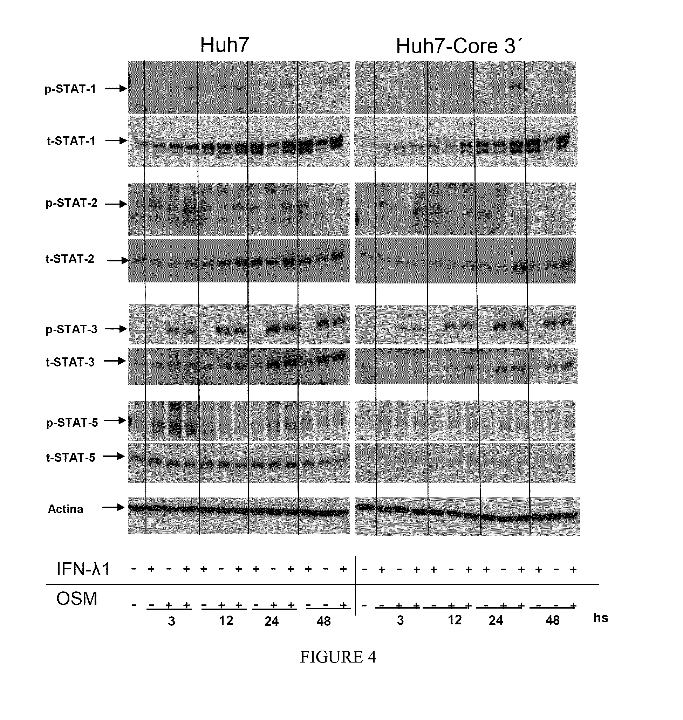

Figure 4. Activation of the Jak-STAT signalling pathway following treatment for 3, 12, 24 or 48 hours with IFN-λ1 (5,000 IU/ml), OSM (10 ng/ml) or both simultaneously administered, in Huh7 cells or Huh7-Core 3' cells carrying the HCV genomic replicon. The legend "+" next to a compound (OSM or IFN-λ1) indicates that the substance to be assayed comprises said compound.

### DETAILED DESCRIPTION OF THE INVENTION

The authors of this invention have demonstrated the capacity of oncostatin M to synergically increase the activity of type III interferons. Specifically, as may be observed in the examples of this invention, OSM synergically enhances the capacity of IFN-λ1 and IFN- λ2 to inhibit the replication of HCV in hepatoma cells (see examples 1 and 2), as well as the capacity of said interferons to promote the activation of genes involved in antigenic presentation in said cells (see example 3).

### COMPOSITIONS OF THE INVENTION

A first aspect of the invention relates to a composition or kit comprising, together or separately,
(i) a first component selected from the group of
   (a) an OSMR agonist and
   (b) a polynucleotide that encodes an OSMR agonist.
(ii) a second component selected from the group of
   (a) at least one type III interferon or a functionally equivalent variant thereof, and
   (b) at least one polynucleotide that encodes a type III interferon or a functionally equivalent variant thereof.

The term "composition", as used in this invention, refers to a composition of matter that comprises the above-mentioned components, that is, an OSMR agonist and at least the type III interferon or a functionally equivalent variant thereof, or the polynucleotides that encode said molecules, as well as any product resulting, directly or indirectly, from the combination of the different components in any quantity thereof. Those skilled in the art will appreciate that the composition may be formulated as a single formulation or may be presented as separate formulations of each of the components, which may be combined for joint used as a combined preparation. The composition may be a kit of parts wherein each of the components is individually formulated and packaged.

The term "protein", which is used interchangeably with polypeptide herein, refers to a chain of amino acids of any length wherein the different amino acids are linked to one another by means of peptide bonds or disulphide bridges.

The term "polynucleotide", as used in this invention, refers to a polymer formed by a variable number of monomers wherein the monomers are nucleotides, including both ribonucleotides and deoxyribonucleotides. The polynucleotides include monomers modified by methylation as well as unmodified forms. The terms "polynucleotide" and "nucleic acid" are used interchangeably in this invention and include mRNA, cDNA and recombinant polynucleotides. As used in this invention, the polynucleotides are not limited to polynucleotides as they appear in nature, but include polynucleotides wherein non-natural nucleotide analogues and internucleotide bonds.

### First component of the compositions of the invention

The first component of the compositions of the invention is selected from the group of
(a) an OSMR agonist and
(b) a polynucleotide that encodes an OSMR agonist.

The term "OSMR agonist", as used in this invention, refers to any ligand, both natural and non-natural, that binds to the polypeptide known as OSMR and which is capable of activating the signalling mediated by at least one of the heterodimeric receptors wherein OSMR participates. Thus, the agonists of the invention are capable of activating the signalling mediated by the receptor formed by OSMR and gp130, the receptor formed by OSMR and IL31RA, and the receptor formed by gp130 and LIFR, said binding leading to an increase in at least one of the biological activities of OSM, including the capacity to inhibit the growth of A375 melanoma cells, as described by Zarling et al. (Proc. Natl. Acad. Sci. USA. 1986, 83:9739-9743), and/or the capacity to inhibit the replication of HCV in hepatoma cells (HepG2 or Huh7), as described by Larrea et al. (J. Virol. 2009, 83:3298-3311), Ikeda et al. (FEBS Letters, 2009, 583:1434-1438) or in examples 1 and 2 of this invention.

Examples of OSMR agonists suitable to be used in this invention include both oncostatin M and any functionally equivalent variant thereof, including the variants disclosed in W09109057.

The term "oncostatin M" or "OSM", as used herein, refers to a pleiotropic IL-6-type cytokine, a glycoprotein with an approximate molecular weight of 28,000, primarily produced by T cells, monocytes/macrophages and dendritic cells, which was originally isolated from a medium conditioned by PMA-treated U-937 human histiocytic leukemia cells based on its ability to inhibit growth of A375 melanoma cells (Zarling, et al.; 1986, Proc. Natl. Acad. Sci. USA, 83:9739-9743; and Brown et al., 1987, J. Immunol. 139:2977-83). OSM also shows the capacity to inhibit the growth of the mouse myeloid leukemia M1 cell line, as well as other solid tumour cells (Grant et al., 1999, Mol. Med. Today 5:406-12; and Gibbs et al., 1998, Melanoma Res. 8: 221-226), as well as the growth-stimulating activity of normal fibroblasts, AIDS-Kaposi's sarcoma cells and a human erythroleukemia cell line, TF-1 (Nair et al., 1992, Science, 255:1430-1432; and Miles et al., 1992, Science 255:1432-1434). Document W02006084092 discloses the use of antibodies that bind to the OSM receptor beta subunit (OSMRβ) to inhibit the growth of cancerous cells.

The cDNA of human OSM encodes the 252-amino-acid pre-pro-OSM, which comprises a 25-residue hydrophobic signal peptide and a 31-amino-acid hydrophilic C-terminal domain that are eliminated during the maturation of OSM to generate the mature 196-residue form of OSM. The accession number of pre-pro-OSM of human origin indexed in the NCBI database is P13725 (SEQ. ID. NO.: 1). The mature form of OSM (SEQ. ID. NO.: 2) corresponds to the region between the alanine residue at position 26 and the arginine residue at position 221, both included.

The invention contemplates the use of OSM variants, which may be both natural and artificial. The expression "natural variant" refers to all those variants of human OSM mentioned above which appear in native form in other species, that is, the orthologues of OSM, and include, without limitation, OSM of bovine origin, the precursor form whereof corresponds to the sequence with accession number P53346 in the NCBI database and the active form whereof corresponds to amino acids 21 to 194 of said sequence, as well as mouse OSM, the precursor form whereof corresponds to the sequence with accession number P53347 in the NCBI database and the active form whereof corresponds to amino acids 21 to 194 of said sequence.

In a preferred embodiment, the first component of the invention corresponds to the mature form of OSM of human origin, which comprises sequence SEQ. ID. NO.: 2, optionally including the post-translational modifications that appear when the above-mentioned sequence is expressed in cells of human origin, such as the disulfur bridge between amino acids 31 and 152, the disulfur bridge between amino acids 74 and 192, and glycosylations at positions 100 and/or 217.

Alternatively, the first component of the invention may be a functionally equivalent variant of oncostatin M of artificial origin. The term "functionally equivalent variant of oncostatin M", as used in this invention, refers to polypeptides the sequence of which is derived from the sequences mentioned above by the addition, deletion or substitution of one or more amino acids, and which substantially conserve the functional properties of OSM, including the capacity to inhibit the growth of A375 melanoma cells, as described by Zarling et al. (Proc. Natl. Acad. Sci. USA 1986, 83:9739-9743), and/or the capacity to inhibit the replication of HCV in hepatoma cells (HepG2 or Huh7), as described by Larrea et al. (Journal of Virology, 2009, 83:3298-3311), Ikeda et al. (FEBS Letters, 2009, 583:1434-1438), or in examples 1 and 2 of this invention.

The variants of oncostatin M considered within the context of this invention include polypeptides that exhibit at least 60%, 65%, 70%, 72%, 74%, 76%, 78%, 80%, 90% or 95% similarity or identity with the different natural variants of oncostatin M mentioned above. The degree of identity between two polypeptides is determined by using computer-implemented algorithms and methods that are widely known by those skilled in the art. The identity between two amino acid sequences is preferably determined using the BLASTP algorithm (BLAST Manual, Altschul, S., et al., NCBI NLM NIH Bethesda, Md. 20894, Altschul, S., et al., J. Mol. Biol., 1990, 215:403-410).

The first component of the invention may also correspond to a nucleic acid that encodes oncostatin M or the functionally equivalent variant thereof. Thus, in case that the nucleic acid encodes OSM, the latter may be of human origin, corresponding to the sequence with accession number NM_020530 in NCBI, of bovine origin, corresponding to the sequence with accession number NM_175713 in NCBI, of mouse origin, corresponding to the sequence with accession number NM_001013365 in NCBI. However, the invention considers the use of all those polynucleotides that encode the variants of OSM mentioned above.

Those skilled in the art will appreciate that the nucleic acid that forms the first component of the invention must be expressed in the interior of a cell and eventually secreted into the medium; to this end, the sequence that encodes oncostatin M or the functionally equivalent variant thereof may exhibit, at one 5'-end, a sequence that encodes a secretory signal. The expression "secretory signal sequence", as used in this invention, refers to an amino acid sequence that is capable of promoting the access of all those proteins that present said sequence at the N-terminal end to the cell secretory pathway. Signal sequences suitable to be used in this invention include, amongst others, the signal sequence of the tissue plasminogen activator (tPA), the growth hormone, GM-CSF and immunoglobulin and, in particular, Igκ or IgVχ. Preferably, the signal sequence that is a part of the first component of the invention is the signal sequence of OSM, corresponding to amino acids 1 to 21 of the sequence identified in SEQ. ID. NO.: 1.

Alternatively, the first component of the invention may be a nucleic acid that exhibits a sequence identity of at least 70%, at least 75%, at least 80%, at least 85%, at least 90%, at least 91%, at least 92%, at least 93%, at least 94%, at least 95%, at least 96%, at least 97%, at least 98% or at least 99% identity with any of the sequences mentioned above, wherein the percentage of identity is determined using an algorithm of the GAP, BESTFIT or FASTA type, the computer implementation whereof appears in the Wisconsin Genetics Software Package Release 7 (Wisconsin Genetics Software Package Release 7.0, Genetics Computer Group, 575 Science Dr., Madison, Wis.) and which use the Smith and Waterman (Adv. Appl. Math., 1981, 2:482), the Needleman and Wunsch (J. Mol. Biol. 1970, 48: 443), or the Pearson and Lipman (Proc. Natl. Acad. Sci. USA, 1988, 85:2444) local algorithms, using the default values for the different parameters.

Alternatively, the first component of the composition of the invention is a polynucleotide that is capable of specifically hybridising with any of the sequences that encode oncostatin M or the variants thereof Within the context of this invention, "polynucleotides capable of specifically hybridising with a target polynucleotide" is understood to mean those polynucleotides that are capable of hybridising under stringent conditions, understanding stringent conditions to mean those conditions that allow for the specific hybridisation of two nucleic acids at temperatures of about 65°C, for example, in a 6X SSC solution, 0.5% SDS, 5% Denhardt's solution and non-specific denatured DNA at a concentration of 100 µg/ml and any other solution with an equivalent ionic strength, and following a washing step at 65°C in the presence of a solution of, for example, 0.2% SSC and 0.1% SDS, and any other solution with an equivalent ionic strength. However, the strict conditions may be adapted by the person skilled in the art on the basis of the size of the sequence to be hybridised, on the basis of the GC content and on the basis of other parameters. Adequate methods for the selection of the adequate hybridisation conditions have been described by Sambrook et al., 2001 (Molecular Cloning: A Laboratory Manual, 3rd Edition, Laboratory Press, Cold Spring Harbor, New York).

### Second component of the compositions of the invention

The second component of the compositions of the invention is selected from the group of
(a) at least one type III interferon or a functionally equivalent variant thereof, and
(b) at least one polynucleotide that encodes a type III interferon or a functionally equivalent variant thereof.

Within the context of the invention, "a type III interferon" refers to any member of the interferon family that is capable of binding to and activating the signalling mediated by the specific heterodimeric receptor, which comprises a first chain called IFNL-R1, IL-28Rα or CRF2-12 that is specific for type III interferon, and a second chain called ILI0R2, IL-10Rβ or CRF2-4 that acts as a shared subunit for other cytokines, such as IL-10, IL-22 or IL-26. The binding and activation of said receptor results in the induction of the ISGF3 complex, the overexpression of class I MHC molecules and the activation by phosphorylation of different members of STATs, specifically, STAT1, STAT2, STAT and STAT5. Similarly, the activation of the signalling mediated by type III IFN results in an inhibition of the proliferation of different cell lines.

Therefore, the term type III interferon includes polypeptides such as those known as interferon (IFN-λ1 or IL-29), interferon λ2 (IFN-λ2 or IL-28A) and interferon λ3 (IFN-X3 or IL-28B), the sequences whereof are described in the GenBank database under accession numbers NP_742152, NP_742150 and NP_742151, respectively.

In a preferred embodiment, the type III interferon is IFN-λ1, also known as IL29 or zcyto21, which corresponds to the human polypeptide encoded by the gene with GeneID 282618, and which encodes a 200-amino-acid precursor (SEQ. ID. NO.: 3; NP_742152.1; UniprotKB: Q8IU54.1). The mature form of said precursor corresponds to a 181-amino-acid polypeptide, corresponding to amino acids 20 to 200 of the precursor, the sequence whereof is identified as SEQ. ID. NO.: 4.

The invention also considers functionally equivalent variants of IFN-λ1, which correspond to all those polypeptides resulting from the substitution, addition or deletion of one or several amino acids from the sequence mentioned above, and substantially conserve one or several functionalities of IFN-λ1, such as the capacity to promote an increase in the expression of class I histocompatibility antigens, the capacity to induce different genes generically known as ISG, which include proteins with antiviral activity, such as double-stranded-RNA-activated serine/threonine protein kinase, protein kinases R (PKR), β2-microglobulin, the 2',5'-OAS protein and the MxA protein.

In the particular case of the functionally equivalent variants of IFN-λ1, these may be characterised by their capacity to protect HT29 cells (colorectal adenocarcinoma), A549 cells (lung carcinoma) and HaCaT (keratinocytes) infected by the mediation of VSV, essentially determined as described in Kotenko et al. (Nat. Immunol., 2003, 4:69-77), as well as to protect hepatocellular carcinoma HepG2 cells from the cytopathogenic effects mediated by EMCV, essentially determined as described by Sheppard et al. (Nat. Immunol., 2003, 4:63-68). However, the expression "functionally equivalent variant of IFN-λ1" specifically excludes any type I interferon. The distinction between a type I interferon and a functionally equivalent variant of IFN-λ1 is performed using the methods described by Kotenko, S.V., et al. (Nat. Immunology, 2003, 1:69-77), as well as by comparing the effect on viral replication in certain viruses that show greater sensitivity to type III IFN than to type I IFN (see Ank, N., et al., J. Virol., 2006, 80:4501-4509).

Functionally equivalent variants of IFN-λ1 suitable to be used in this invention essentially include the variants disclosed in WO2009/149377. Specific variants include the following (wherein the positions that are modified are indicated by taking the mature 181-amino-acid protein as a reference):
- Variants that comprise a methionine residue, a dipeptide with sequence ML, MI, or a tetrapeptide with sequence MEAE at the N-terminal position;
- Variants at position 10, specifically, variants with substitution T10P;
- Variants at position 15, specifically, variants C15K, C15N, C15R, C15S, C15T, C15I, C15M, C15E, C15D, C15G, C15A, C15V, C15Y, C15W, C15L or C15F;
- Variants at position 18, specifically, variants G18D and G18E;
- Variants at position 169, specifically, variant N169D;
- Variants at position 171, specifically, variants C171K, C171N, C171R, C171S, C171T, C171I, C171M, C171E, C171D, C171G, C171A, C171V, C171Y, C171W, C171L or C171F;
- Variants that contain a deletion of 1, 2, 3, 4, 5 or 6 amino acids at the N-terminal end or at the C-terminal end;
- Variant Δ1-6/C171S (SEQ. ID. NO.: 5), which, optionally, may present a methionine at the N-terminal position;
- Recombinant IFN-λ1, which presents the sequence specified in SEQ. ID. NO.: 6;
- Conjugates of IFN-λ1 with the constant region of an immunoglobulin, similar to those described for IFN-β in W006000448;
- Conjugates of IFN-λ1 with ApoA, as described in W02009150284;
- Conjugates of IFN-λ1 with albumin, as described for type I IFN in Rustgi, V. (Curr. Med. Res. Opin., 2009, 25:991-1002), in W005077042 and in W0200833413.
- Variants of any of the above obtained by the conjugation of IFN-λ1 with a hydrosoluble polymer of the types polyethylene glycol (PEG), monomethoxy-PEG, mono-(C1-C10)alkoxy-PEG, aryloxy-PEG, poly-(N-vinyl pyrrolidone)-PEG, tresylmonomethoxy-PEG, monomethoxy-PEG propionaldehyde, PEG propionaldehyde, bis-succinimidyl carbonate PEG, homopolymers of propylene glycol, a copolymer of a polypropylene oxide/ethylene oxide, polyoxyethylated polyols (e.g. glycerol), monomethoxy-PEG butyraldehyde, PEG butyraldehyde, monomethoxy-PEG acetaldehyde, PEG acetaldehyde, methoxy PEG-succinimidyl propionate, methoxy PEG-succinimidyl butanoate, polyvinyl alcohol, dextran, cellulose or other carbohydrate-based polymers. PEGs suitable to be conjugated with the variants of IFN-λ1 include PEG with a molecular weight of about 600 to about 60,000, including, for example, 5,000 daltons, 12,000 daltons, 20,000 daltons, 30,000 daltons and 40,000 daltons, which may be linear or branched. Preferably, the pegylated IFN-λ1 is obtained by pegylation with a 20,000-dalton PEG-propionaldehyde essentially obtained as described in W02007041713.

Other variants of IFN-λ1 considered within the context of this invention include polypeptides that exhibit at least 60%, 65%, 70%, 72%, 74%, 76%, 78%, 80%, 90% or 95% similarity or identity with the different natural variants of IFN-λ1 mentioned above. The degree of identity between two polypeptides is determined using algorithms such as those described above within the context of oncostatin M.

In a preferred embodiment, IFN-λ1 corresponds to the molecule known as PEG-rIL-29, which is currently in clinical trials and corresponds to Δ6-IFN-λ1 modified at the N-terminal end with a methionine residue and modified by pegylation with 20,000-dalton PEG-propionaldehyde (SEQ. ID. NO.: 7).

The type III interferon that forms the second component of the invention may also correspond to a nucleic acid that encodes IFN-λ1 or any of the functionally equivalent variants thereof mentioned above. Thus, in case that the nucleic acid encodes IFN-λ1, the latter may be of human origin, corresponding to the sequence with accession number AY129150 in NCBI, of feline origin *(Felis catus),* corresponding to the sequence with accession number AB241610 in NCBI, of porcine origin *(Sus scrofa),* corresponding to the sequence with accession number FJ455508 in NCBI, or of canine origin *(Canis lupus),* corresponding to the sequence with accession number AB241609 in NCBI.

Alternatively, the second component of the invention may be a nucleic acid that shows a sequence identity of at least 70%, at least 75%, at least 80%, at least 85%, at least 90%, at least 91%, at least 92%, at least 93%, at least 94%, at least 95%, at least 96%, at least 97%, at least 98% or at least 99% identity with any of the sequences mentioned above, wherein the percentage of identity is essentially determined as described above within the context of the first component of the invention. Likewise, the second component of the composition of the invention is a polynucleotide that is capable of specifically hybridising with any of the sequences that encode IFN-λ1 or the variants thereof, wherein the "polynucleotides capable of specifically hybridising with a target polynucleotide" are defined in the same manner as those mentioned above within the context of the first component of the invention.

In another preferred embodiment, the type III interferon is IFN-λ2, also known as IL28A or zcyto20, which corresponds to the polypeptide encoded by the human gene with GeneID 28261 and produces a 200-amino-acid precursor (SEQ. ID. NO.: 8; NP_742150.1; UniprotKB: Q8IZJ0.1) that is processed in order to produce the mature 175-amino-acid protein, corresponding to amino acids 26 to 200 of SEQ. ID. NO.: 8, and which is represented by SEQ. ID. NO.: 9.

The invention also considers functionally equivalent variants of IFN-λ2, which correspond to all those polypeptides resulting from the substitution, addition or deletion of one or several amino acids from the sequence mentioned above and which substantially conserve the capacity of IFN-λ2 to promote an increase in the expression of class I histocompatibility antigens, as well as the induction of different genes generically known as ISG, which include proteins with antiviral activity such as double-stranded-RNA-activated serine/threonine protein kinase, protein kinases R (PKR), β2-microglobulin, the 2',5'-OAS protein and the MxA protein. In the particular case of functionally equivalent variants of IFN-λ2, these may be characterised by their capacity to protect hepatocellular carcinoma HepG2 cells from the cytopathogenic effects mediated by EMCV, essentially determined as described by Sheppard et al. (Nat. Immunol., 2003, 4:63-68), as well as their capacity to reduce the cytopathogenic effects caused by infection with HBV or HCV in Huh7 cells, determined as described by Doyle et al. (Hepatology, 2006, 44:896-906). The distinction between a type I interferon and a functionally equivalent variant of IFN-λ2 is performed using the methods described by Kotenko, S.V., et al. (Nat. Immunology 2003, 1:69-77), as well as by comparing the effect on viral replication in certain viruses that show greater sensitivity to type III IFN than to type I IFN (see Ank, N., et al., J. Virol., 2006, 80:4501-4509).

Preferred functionally equivalent variants of IFN-λ2 include the following (the numbering of the residues is indicated in relation to the position in SEQ. ID. NO.: 9):
- Variants that present a methionine residue at the N-terminal position;
- Variants that present a substitution in the residue at position 48, including C48K, C48N, C48R, C48S, C48T, C48I, C48M, C48E, C48D, C48G, C48A, C48V, C48Y, C48W, C48 L or C48 F;
- Variants that present a substitution in the residue at position 50, including C50K, C50N, C50R, C50S, C50T, C50I, C50M, C50E, C50D, C50G, C50A, C50V, C50Y, C50W, C50L or C50F;
- Variants that lack the amino acid at the C-terminal position (IFN- λ2-Δ175);
- Combinations of any of the above, preferably IFN- λ2 C48S or IFN-λ2 C50S, modified at the N-terminal end by the presence of a methionine residue;
- Recombinant IFN-λ2 with the sequence SEQ. ID. NO.: 10;
- Conjugates of IFN-λ2 with other polypeptides, such as the Fc region of immunoglobulins, albumin or ApoA, or with hydrosoluble polymers such as those described above within the context de IFN-λ1, including pegylated variants of IFN-λ2.

Other variants of IFN-λ2 considered within the context of this invention include polypeptides that exhibit at least 60%, 65%, 70%, 72%, 74%, 76%, 78%, 80%, 90% or 95% similarity or identity with the different natural variants of IFN-λ2 mentioned above. The degree of identity between two polypeptides is determined using algorithms such as those described above within the context of oncostatin M.

The type III interferon that forms the second component of the invention may also correspond to a nucleic acid that encodes IFN-λ2 or any of the functionally equivalent variants thereof mentioned above. Thus, in case that the nucleic acid encodes IFN-λ2, the latter may be of human origin, corresponding to the sequence with accession number AY129148 in NCBI, of mouse origin, corresponding to the sequence with accession number AY869695 in NCBI.

Alternatively, the second component of the invention may be a nucleic acid that shows a sequence identity of at least 70%, at least 75%, at least 80%, at least 85%, at least 90%, at least 91%, at least 92%, at least 93%, at least 94%, at least 95%, at least 96%, at least 97%, at least 98% or at least 99% identity with any of the sequences mentioned above, wherein the percentage of identity is essentially determined as described above within the context of the first component of the invention. Similarly, the second component of the composition of the invention is a polynucleotide that is capable of specifically hybridising with any of the sequences that encode IFN-λ2 or the variants thereof, wherein the "polynucleotides capable of specifically hybridising with a target polynucleotide" are defined in the same manner as those mentioned above within the context of the first component of the invention

In another preferred embodiment, the type III interferon is IFN-λ3 , also known as IL28B or zcyto22, which corresponds to the polypeptide encoded by the human gene with GeneID 282617 and produces a 200-amino-acid precursor (SEQ. ID. NO.: 11, NP_742151.2; UniprotKB: Q8IZI9-1) that is processed in order to produce the mature 175-amino-acid protein, corresponding to amino acids 26 to 200 of SEQ. ID. NO.: 11, the sequence whereof is specified by SEQ. ID. NO.: 12).

The invention also encompasses functionally equivalent variants of IFN-λ3, which correspond to all those polypeptides resulting from the substitution, addition or deletion of one or several amino acids from the sequence mentioned above and which substantially conserve the capacity of IFN-λ3 to promote an increase in the expression of class I histocompatibility antigens, as well as the induction of different genes generically known as ISG, which include proteins with antiviral activity such as double-stranded-RNA-activated serine/threonine protein kinase, protein kinases R (PKR), β2-microglobulin, the 2',5'-OAS protein and the MxA protein. The distinction between a type I interferon and a functionally equivalent variant of IFN-λ3 is performed using the methods described by Kotenko, S.V., et al. (Nat. Immunology 2003, 1:69-77), as well as by comparing the effect on viral replication in certain viruses that show greater sensitivity to type III IFN than to type I IFN (see Ank, N., et al., J. Virol., 2006, 80:4501-4509).

Preferred functionally equivalent variants of IFN-λ3 include the following (the numbering of the residues is indicated in relation to the position in SEQ. ID. NO.: 12):
- Variants that present a methionine residue at the N-terminal position;
- Variants that present a substitution in the residue at position 48, including C48K, C48N, C48R, C48S, C48T, C48I, C48M, C48E, C48D, C48G, C48A, C48V, C48Y, C48W, C48 L or C48 F;
- Variants that present a substitution in the residue at position 50, including C50K, C50N, C50R, C50S, C50T, C50I, C50M, C50E, C50D, C50G, C50A, C50V, C50Y, C50W, C50L or C50F;
- Variants that present a substitution in the residue at position 87 and, in particular, T87S;
- Variants that present a substitution in the residue at position 135 and, in particular, H135Y;
- Variants that lack the amino acid at the C-terminal position (IFN-λ3-Δ175);
- Combinations of any of the above, modified at the N-terminal end by the presence of a methionine residue;
- Conjugates of IFN-λ3 with other polypeptides, such as the Fc region of immunoglobulins, albumin or ApoA, or with hydrosoluble polymers such as those described above within the context of IFN-λ1, including pegylated variants of IFN-λ3.

Other variants of IFN-λ3 considered within the context of this invention include polypeptides that show at least 60%, 65%, 70%, 72%, 74%, 76%, 78%, 80%, 90% or 95% similarity or identity with the different natural variants of IFN-λ3 mentioned above. The degree of identity between two polypeptides is determined using algorithms such as those described above within the context of oncostatin M.

The type III interferon that forms the second component of the invention may also correspond to a nucleic acid that encodes IFN-λ3 or any of the functionally equivalent variants thereof mentioned above. Thus, in case that the nucleic acid encodes IFN-λ3, the latter may be of human origin, corresponding to the sequence with accession number AY129149 in NCBI, of mouse origin, corresponding to the sequence with accession number AY184375 in NCBI, of porcine origin, corresponding to the sequence with accession number GQ996936 in NCBI, or of canine origin, corresponding to the sequence with accession number XM_850273.1 in NCBI.

Alternatively, the second component of the invention may be a nucleic acid that shows a sequence identity of at least 70%, at least 75%, at least 80%, at least 85%, at least 90%, at least 91%, at least 92%, at least 93%, at least 94%, at least 95%, at least 96%, at least 97%, at least 98% or at least 99% identity with any of the sequences mentioned above, wherein the percentage of identity is essentially determined as described above within the context of the first component of the invention. Likewise, the second component of the composition of the invention is a polynucleotide that is capable of specifically hybridising with any of the sequences that encode IFN-λ3 or the variants thereof, wherein the "polynucleotides capable of specifically hybridising with a target polynucleotide" are defined in the same manner as those mentioned above within the context of the first component of the invention.

Alternatively, the first and the second component of the invention may be found as a part of a single molecule. Thus, in case that the first and the second component of the composition are polypeptides, said single molecule is a fusion protein that comprises: (i) an OSMR agonist, and (ii) a type III interferon or a functionally equivalent variant thereof.

The term "fusion protein", as used in this invention, refers to polypeptides that comprise two or more regions from different or heterologous proteins.

Alternatively, in case that both the first and the second component of the composition are polynucleotides, said single molecule is a polynucleotide that encodes a fusion protein comprising: (i) an OSMR agonist, preferably OSM or a functionally equivalent variant thereof, and (ii) a type III interferon or a functionally equivalent variant thereof, or a polynucleotide that contains regions that encode: (i) an OSMR agonist, preferably OSM or a functionally equivalent variant thereof, and (ii) a type III interferon or a functionally equivalent variant thereof.

In this case, when the first and the second component are polypeptides, the invention contemplates compositions wherein the first component is located at the N-terminal position with respect to the second component, and compositions wherein the first component is located at the C-terminal position with respect to the second component.

In case that the first and the second component are of polynucleotides, the invention contemplates compositions wherein the first component is located at the 5'-position with respect to the second component and compositions wherein the first component is located at the 3'-position with respect to the second component.

In both cases, the first and the second component may be directly associated, that is, the C-terminal end of the first component may be associated with the N-terminal end of the second component, or the C-terminal end of the second component may be associated with the N-terminal end of the first component, or the 3'-end of the first component may be associated with the 5'-end of the second component, and compositions wherein the 3'-end of the second component is associated with the 5'-end of the first component.

Alternatively, another aspect of the invention refers to compositions wherein the fusion of the first and the second component is performed through a peptide linker (in case that the first and the second component are of polypeptide character) or a sequence that encodes a peptide linker (in case that the first and the second component are of polynucleotide character). Said peptide linker comprises at least two amino acids, at least three amino acids, at least five amino acids, at least ten amino acids, at least 15 amino acids, at least 20 amino acids, at least 30 amino acids, at least 40 amino acids, at least 50 amino acids, at least 60 amino acids, at least 70 amino acids, at least 80 amino acids, at least 90 amino acids or approximately 100 amino acids. Linkers suitable to be used in this invention are all those that have been described above as suitable to bind two polypeptides and which allow for each of the polypeptides to conserve their native structure, such as those disclosed in document W02009150284.

In those cases wherein the first or the second component of the invention is a polynucleotide or wherein both components are a part of a single polypeptide chain that encodes a fusion protein or contains regions that encode an OMSR agonist and a type III interferon, said polynucleotide may be operatively associated with an expression regulatory region, thereby leading to a gene construct. In principle, any regulatory region may be used for the gene constructs of this invention, provided that said regulatory region is compatible with the cells wherein the polynucleotide is to be expressed. Thus, suitable promoters for the embodiment of this invention include, without being necessarily limited thereto, constitutive promoters such as those derived from the genomes of eukaryotic viruses such as the polyoma virus, adenovirus, SV40, CMV, avian sarcoma virus, hepatitis B virus, the promoter of the methallothionein gene, the promoter of the thymidine kinase gene of the herpes simplex virus, LTR regions of retroviruses, the promoter of the immunoglobulin gene, the promoter of the actin gene, the promoter of the EF-1-alpha gene, as well as inducible promoters, wherein the expression of the protein is dependent on the addition of a molecule or an exogenous signal, such as the tetracycline system, the NFκB/UV light system, the Cre/Lox system and the promoter of thermal shock genes, the regulateable promoters of RNA polymerase II described in WO/2006/135436, as well as tissue-specific promoters. Since the activity of the compositions of the invention is primarily manifested in hepatic cells, said regulatory region may be a liver-specific regulatory region and, more specifically, a liver-specific promoter. Suitable liver-specific promoters include, without limitation, a promoter of al-anti-trypsin (AAT), a promoter of thyroid-hormone-binding globulin, a promoter of alpha-fetoprotein, a promoter of alcohol dehydrogenase, a promoter of IGF-II, the promoter of factor VIII (FVIII), a basic core promoter (BCP) of HBV and PreS2 promoter, a promoter of albumin, a promoter of thyroxine-binding globulin (TBG), a hybrid promoter of the hepatic control region (HCR)-ApoCII, an HCR-hAAT hybrid promoter, an AAT promoter combined with the enhancing element of the mouse albumin gene (Ealb), a promoter of apolipoprotein E, a promoter of low-density lipoprotein, a promoter of pyruvate kinase, a promoter of phosphoenolpyruvate carboxykinase, a promoter of lecithin-cholesterol acyl transferase (LCAT), a promoter of apolipoprotein H (ApoH), the transferrin promoter, a promoter of transthyretin, promoters of alpha-fibrinogen and beta-fibrinogen, a promoter of alpha-1-antichymotrypsin, a promoter of alpha-2-HS glycoprotein, a haptoglobin promoter, a ceruloplasmin promoter, a plasminogen promoter, promoters of complement proteins (CIq, CIr, C2, C3, C4, C5, C6, C8, C9, and complement factor I and factor H), promoter of the complement C3 activator and of the α1-acid glycoprotein. Additional tissue-specific promoters may be found in Tissue-Specific Promoter Database, TiProD (Nucleic Acids Research, J4:D104-D107 (2006)).

### THERAPEUTIC COMPOSITIONS OF THE INVENTION

The compositions of the invention are useful for the treatment of diseases that require treatment with a type III interferon. Therefore, another aspect of the invention relates to a pharmaceutical preparation comprising a therapeutically effective quantity of a composition of the invention and a pharmaceutically acceptable carrier or excipient.

Preferred excipients to be used in this invention include sugars, starches, celluloses, rubbers and proteins. In a particular embodiment, the pharmaceutical composition of the invention will be formulated in a pharmaceutical form designed for solid administration (for example, tablets, capsules, pills, granules, suppositories, crystalline or amorphous sterile solids that may be reconstituted to provide liquid forms, etc.), liquid administration (for example, solutions, suspensions, emulsions, elixirs, lotions, ointments, etc.) or semi-solid administration (gels, ointments, creams and similar). The pharmaceutical compositions of the invention may be administered by any route, including, without being limited thereto, oral, intravenous, intramuscular, intraarterial, intramedullary, intrathecal, intraventricular, transdermal, subcutaneous, intraperitoneal, intranasal, enteric, topical, sublingual or rectal. A review of the different forms of administration of active principles, of the excipients to be used and the methods for the manufacturing thereof may be found in Tratado de Farmacia Galénica, C. Faulí i Trillo, Luzán 5, S.A. de Ediciones, 1993, and in Remington's Pharmaceutical Sciences (A.R. Gennaro, Ed.), 20th edition, Williams & Wilkins PA, USA (2000). Examples of pharmaceutically acceptable carriers are known in the state of the art and include phosphate-buffered saline solutions, water, emulsions, such as oil/water emulsions, different types of wetting agents, sterile solutions, etc. The compositions that comprise said vehicles may be formulated by conventional methods known in the state of the art.

The formulations designed for parenteral administration may be in the form of aqueous or non-aqueous solutions or suspensions for sterile isotonic injections. For example, injectable solutions may be prepared wherein the carrier comprises saline solution, glucose solution or a mixture of saline and glucose solutions. Injectable suspensions may also be prepared; in this case, suitable liquid carriers, suspension agents and similar may be used. They also include solid-form preparations that are transformed, right before they are to be used, into liquid-form preparations. In those compositions suitable for percutaneous administration, the carrier optionally comprises a suitable penetration-enhancing agent and/or wetting agent, optionally combined with suitable additives of any type in lower proportions; these additives do not introduce a significant harmful effect on the skin. Suitable additives may be antioxidant agents, preservatives, stabilising agents, emulsifiers, salts designed to affect the osmotic pressure and/or buffer substances.

Alternatively, the compositions of the invention may be released to the target cells if the composition is formulated in liposomes. The liposomes may be prepared using conventional techniques, including sonication, dialysis with chelates, homogenisation, infusion of solvent coupled to extrusion, extrusion by freezing-thawing, microemulsification and others. The reagents used to cross-link a liposome or another agent that contains lipids to the proteins of this invention comprise a phospholipid derivative, to be anchored at one end of the cross-linking in the lipid layer, and a reactive group at the other end, to provide a binding point for the target biomolecule. Polymerised liposomes or polymer-coated liposomes may also be used. Said polymers may stabilise the liposome, reduce their purging from the body and/or reduce their immunogenicity. The liposome may also be loaded with a functional group, such as a diagnostic or therapeutic agent, during or after the formation thereof The agent may be contained in an aqueous core of the liposome or may be incorporated into or bound to the surrounding membrane thereof.

Alternatively, the compositions of the invention may be formulated in the form of prolonged-release preparations. Suitable examples of prolonged-release preparations include semi-permeable matrices of solid hydrophobic polymers that contain the composition of the invention, wherein the matrices are in the form of molded items, for example, films or microcapsules. Examples of prolonged-release matrices include polyesters, hydrogels (for example, poly(2-hydroxyethyl methacrylate), or poly(vinyl alcohol)), polylactides (U.S. patent no. 3.773.919), copolymers of L-glutamic acid and ethyl L-glutamate, non-degradable ethylene-vinyl acetate, degradable lactic acid-glycolic acid copolymers, such as LUPRON DEPOTTM (injectable microspheres composed of lactic acid-glycolic acid copolymer and leuprolide acetate), and poly-D-(-)-3-hydroxybutyric acid. Although polymers such as ethylene-vinyl acetate and lactic acid-glycolic acid allow for the release of molecules for over 100 days, certain hydrogels release proteins for shorter periods of time.

In case that the pharmaceutical composition of the invention comprises nucleic acids (the polynucleotides of the invention, the vectors or the gene constructs), the invention considers pharmaceutical compositions especially prepared for the administration of said nucleic acids. The pharmaceutical compositions may comprise said nucleic acids in naked form, that is, in the absence of compounds that protect the nucleic acids from degradation by the body's nucleases, which has the advantage of eliminating the toxicity associated with the reagents used for the transfection. Suitable administration routes for the naked compounds include intravascular, intratumoural, intracraneal, intraperitoneal, intrasplenic, intramuscular, subretinal, subcutaneous, via mucous membranes, topical and oral (Templeton, 2002, DNA Cell Biol. 21:857-867). Alternatively, the nucleic acids may be administered as a part of liposomes, conjugated with cholesterol or conjugated with compounds capable of promoting translocation through the cell membranes, such as the Tat peptide derived from the TAT protein of HIV-1, the third helix of the homeodomain of the Antennapedia protein of D. melanogaster, the VP22 protein of the herpes simplex virus, arginine oligomers and peptides such as those described in WO07069090 (Lindgren, A., et al., 2000, Trends Pharmacol. Sci. 21:99-103; Schwarze, S.R., et al., 2000, Trends Pharmacol. Sci. 21:45-48; Lundberg, M., et al., 2003, Mol. Therapy 8:143-150; and Snyder, E.L., and Dowdy, S.F., 2004, Pharm. Res. 21:389-393). Alternatively, the polynucleotide may be administered as a part of a plasmid vector or a viral vector, preferably vectors based on adenoviruses, adenoassociated viruses or retroviruses, such as viruses based on the murine leukemia virus (MLV) or lentiviruses (HIV, FIV, EIAV).

Those skilled in the art will appreciate that the compositions of the invention may have different proportions of both components and that said proportion will be dependent on the first and the second component used in each particular case, as well as the desired indication. Thus, the invention considers compositions wherein the ratio between the quantities of the two components may range between 50:1 and 1:50, in particular between 20:1 and 1:20, between 1:10 and 10:1, or between 5:1 and 1:5.

### THERAPEUTIC USES OF THE COMPOSITIONS AND FUSION PROTEINS OF THE INVENTION

The compositions of the invention allow obtaining a greater effect than that observed when type III interferon is used individually. Therefore, the compositions of the invention are useful for the treatment of those disorders that require or respond to treatment with type III interferon. Taking into consideration that type III interferons are capable of inhibiting the replication of the HCV replicon in hepatic epithelial cells (see examples 1 and 2) and promoting the expression of genes involved in antigen processing and presentation (see example 3), the compositions of the invention may be used for the treatment of those disorders that require an increase in the immune response against a given antigen, in particular infectious diseases. On the other hand, since the capacity of oncostatin M to reduce the proliferation of melanoma cells is well known (see Zarling et al., Proc. Natl. Acad. Sci. USA, 1986, 83:9739-9743), the compositions of the invention would also be adequate for the treatment of neoplastic diseases and, in particular, melanoma.

Therefore, in another aspect, the invention relates to the compositions of the invention for use in medicine. Another aspect of the invention relates to a composition of the invention for use in the treatment of an infectious or neoplastic disease. Alternatively, the invention relates to the use of a composition of the invention for the manufacture of a medicament for the treatment of an infectious or neoplastic disease. Alternatively, the invention relates to a method for the treatment of an infectious or neoplastic disease in a subject, which comprises the administration to said subject of a composition of the invention.

The infectious or neoplastic diseases that may be treated with the compositions of the invention preferably include diseases that affect epithelial cells or hepatic cells.

The expression "infectious disease", as used in this invention, refers to a disease caused by a pathogen, including, without limitation, diseases caused by a virus, a bacterium, a fungus or a protozoa.

The cellular pathogen infecting the epithelial cell is meant to include pathogenic viruses, bacteria, fungi and parasites, particularly retroviruses, adenoviruses, papiloma viruses, herpes viruses, cytomegaloviruses, adeno-associated viruses, hepatitis viruses, pneumoviruses, noroviruses, rotaviruses, astroviruses, paramixoviruses, mumps viruses, viral proteins, viroids, and similar; meningococchi, mycobacteria, Salmonella sp., Shigella sp., Staphylococcus sp., Campylobacter jejuni, Clostridium sp., Escherichia coli, Yersinia sp., Bordetella pertussis, Treponema sp., and similar; parasites such as Leishmania sp., Toxoplasma sp., Schistosoma sp., Clonorchis sp., or Plasmodium sp., etc.; or fungi such as Histoplasma sp., Aspergillus sp., Candida sp., Cryptococcus sp., Pneumocystis sp., and the like.

In a particular embodiment, the infectious diseases that are treated with the compounds of this invention comprises infectious diseases that affect the liver, such as viral hepatitis, including hepatitis A, hepatitis B, chronic Hepatitis B, hepatitis C, chronic hepatitis C, hepatitis D, hepatitis E and hepatitis X, bacterial infections such as amoeba abscesses, disseminated coccidiomycosis, infections by Francisella sp. and, in particular, *Francisella tularensis,* infections by Plasmodium and, specifically, the hepatic phase of malaria. In a preferred embodiment, the compositions of the invention are used for the treatment of infections caused by HCV.

In the particular case that the compositions of the invention are used for the treatment of HCV, they may be used jointly with other compounds useful for the treatment of hepatitis. Suitable compounds to be used in combination with the compositions of the invention include, without limitation, HCV protease inhibitors, HCV metalloprotease inhibitors, HCV serine-protease inhibitors, HCV RNA polymerase inhibitors, HCV helicase inhibitors, ribavirin (e.g., Pegasys(R); Copegus(R) (Roche), specific anti-HCV monoclonal antibodies, anti-sense compounds, small antiviral molecules, ribozymes and combinations of the above. Some compounds with the capacity to inhibit NS3/4A protease which may be administered by oral route include telaprevir/VX-950 (Vertex/J and J) and Boceprevir/SCH503034 (Schering-Plough). Other inhibitors found in clinical trials include macrocyclic inhibitors of NS3/4A protease, such as ITMN-191/R7227 (Intermune/Roche), BI-201335 (Boehringer Ingelheim), TMC435350 (Medivir/J and J) and MK7009 (Merck); NS5B protease inhibitors, such as R7128 (Pharmasset/Roche), VCH-759 (ViRochem Pharma), PF-00868554 (Pfizer), ANA-598 (Anadys); and NS5A inhibitors such as BMS-790052 (Bristol-Myers Squibb).

The effect of the compositions of the invention on hepatic diseases may be monitored by measuring the hepatic function, wherein said hepatic function refers to the normal functions of the liver, including, without limitation, a synthesis function such as the synthesis of serum proteins (for example, albumin, clotting factors, alkaline phosphatase, aminotransferases such as alanine transaminase, aspartate transaminase, 5'-nucleotidase, γ-glutaminyltranspeptidase, etc.), synthesis of bilirubin, synthesis of cholesterol and synthesis of biliary acids; metabolic functions such as carbohydrate metabolism, amino acid and ammonium metabolism, hormone metabolism and lipid metabolism; xenobiotic detoxification; hemodynamic functions such as spleen and portal hemodynamics, and similar.

The compositions of the invention are capable of inhibiting the replication of the HCV replicon in epithelial hepatic cells (see examples 1 and 2), promoting the expression of genes involved in antigen processing and presentation (see example 3) and inhibiting the proliferation of melanoma cells (Zarling et al., *supra.).* These effects make it possible to use the compositions of the invention in the treatment of neoplastic diseases.

The expression "neoplastic disease", as understood in this invention, refers to any disease characterised by an abnormal growth of cells, both benign (non-cancerous) and malignant (cancerous), and includes both solid and liquid tumours.

The expression "cancer treatment", as used in this invention, is understood to mean all those treatments that achieve a reduction in the size of the tumour, a decrease in the rate of growth of the tumour, a decrease in the migration of the tumour, a decrease in the epithelial-mesenchymal transition, the stabilisation of the size of the tumour, a reduction in the invasive capacity of the tumour, a decrease in the rate of progression of the tumour from one stage to the next, a decrease in the number of metastases, as well as regression of the tumour.

Preferably, the neoplastic diseases that may be treated with the compositions of this invention are those caused by an undesireable proliferation of epithelial cells. The tumour epithelial cells may be selected from carcinomas, such as adenocarcinoma (bronchiolo-alveolar adenocarcinoma, clear-cell adenocarcinoma, folicular adenocarcinoma, mucinous adenocarcinoma, papillary adenocarcinoma, en cuirasse adenocarcinoma, sebaceous adenocarcinoma, adrenocortical adenocarcinoma, carcinoid tumour, acinar cell carcinoma, adenoid cystic carcinoma, ductal carcinoma, endometroid carcinoma, pancreatic adenocarcinoma, gastric carcinoma, colorectal cancer, hepatocellular carcinoma, non-infiltrating intraductal carcinoma, islet cell carcinoma, lobular carcinoma, mucoepidermoid carcinoma, neuroendocrine carcinoma, renal cell carcinoma, signet ring cell carcinoma, cutaneous appendage carcinoma, cholangiocarcinoma, choriocarcinoma, cystadenocarcinoma, Klatskin tumour, extramammary Paget's disease), adenosquamous carcinoma; basal cell carcinoma, basosquamous carcinoma; Ehrlich tumour carcinoma; giant cell carcinoma; *in situ* carcinoma (cervical intraepithelial neoplasia and prostatic intraepithelial neoplasia); Krebs 2 carcinoma; large cell carcinoma; Lewis lung carcinoma; non-small cell lung carcinoma; papillary carcinoma; squamous cell carcinoma (Bowen's disease); transitional cell carcinoma; verrucous carcinoma.

The tumour epithelial cells may also be selected from adnexal and skin appendage neoplasms, such as sebaceous adenocarcinomas, skin appendage carcinoma; basal cell neoplasms, such as basal cell carcinomas (basal cell nevus syndrome), basosquamous carcinoma and pilomatrixoma; cystic, mucinous and serous neoplasms, such as mucinous adenocarcinomas, mucoepidermoid carcinoma, signet ring cell carcinoma / Krukenberg tumour), cystadenocarcinoma (mucinous cystadenocarcinoma, papillary cystadenocarcinoma, serous cystadenocarcinoma), cystadenoma (mucinous cystadenoma, papillary cystadenoma, serous cystadenoma), mucoepidermoid tumour and peritoneal pseudomixoma, ductal, lobular and medullary neoplasms, such as ductal carcinoma (mammary ductal carcinoma, pancreatic ductal carcinoma), non-infiltrating intraductal carcinoma (mammary Paget's disease), lobular carcinoma, medullary carcinoma, extramammary Paget's disease, intraductal papiloma; fibroepithelial neoplasms, such as adenofibroma, Brenner tumour; mesothelial neoplasms, such as adenomatoid tumour, mesothelioma (cystic mesothelioma); and squamous cell neoplasms, such as acanthoma, papillary carcinoma, squamous cell carcinoma (Bowen's disease), verrucous carcinoma, papiloma (inverted papiloma).

In another preferred embodiment, the compositions of the invention are used for the treatment of melanoma.

The term "melanoma", as used in this invention, refers to any tumour resulting from the proliferation of melanocytes that predominantly appear on the skin, but also in the eye or the intestine, and includes, without limitation, melanomas, metastatic melanomas, melanocarcinomas, melanoepitheliomas, melanosarcomas, melanoma *in situ*, superficially-extended melanoma, modular melanoma, malignant lentigo melanoma, acral lentiginous melanoma, invasive melanoma, familial atypical mole and melanoma syndrome (FAM-M).

In a preferred embodiment, the compositions of the invention are primarily used for the treatment of diseases associated with an undesireable proliferation of hepatic cells, including both hyperplasias (focal nodular hyperplasia, nodular regenerative hyperplasia) and hepatic tumours. The term "hepatic tumour", as used in this invention, is understood to be any tumour that originates in the liver and includes both benign tumours, of the hepatocellular adenoma and cavernous hemangioma types, and malignant tumours, such as hepatoblastoma (HB) and hepatocellular carcinoma (CHC). However, the compositions of the invention may also be used for the treatment of other hepatic tumours different from the above, including cholangiocarcinoma, mixed tumours and mesenchymal tissue tumours.

The compositions of the invention may be administered in doses of less than 10 mg per kilogram of body weight, preferably less than 5, 2, 1, 0.5, 0.1, 0.05, 0.01, 0.005, 0.001, 0.0005, 0.0001, 0.00005 or 0.00001 mg for every kg of body weight. The unit dose may be administered by injection, by inhalation or by topical administration. The compositions of the invention may be directly administered in the organ wherein the disease to be treated appears, in which case the doses administered are between 0.00001 mg and 3 mg per organ, or preferably between 0.0001 and 0.001 mg per organ, about 0.03 and 3.0 mg per organ, about 0.1 and 3.0 mg per organ, or between 0.3 and 3.0 mg per organ.

The expressions "international units" and "units" are used interchangeably in this invention to refer to the parameter used to quantify the capacity of interferon to inhibit the cytopathic effect of a specific virus (for example, the encephalomyocarditis virus, the vesicular stomatitis virus or the Semliki forest virus) following the infection of an adequate cell line (for example, the A549 pulmonary carcinoma cell lines; the HEP2/C cell lines and similar). Typically, the activity of the preparation is determined using the method described by Rubinstein et al. (J. Virol., 1981, 37:755-758), wherein 1 unit of interferon is defined as the inverse of the dilution of the preparation comprising interferon necessary to reduce the cytopathic effect of the vesicular stomatitis virus in a reference cell line (MDBK) by 50%.

In the case of type III interferons, although there is no standard assay to determine the activity of interferon, it is possible to define the activity of interferon as the inverse of the dilution of the problem sample that is capable of reducing the cytopathic effect (measured by the formation of plaques in monolayer cultures) in the HepG2 cell line infected with the encephalomyocarditis virus by 50%, as described by Sheppard, P., et al., (Nature Immunol., 2003, 4:63). The value of ED₅₀ to achieve this effect is 1-5 ng/ml for IFN-λ1 and 10-50 ng/ml for IFN-λ2.

Typically, the antiviral activity is normalised by reference to the activity of a standard, such as interferon-alpha, provided by the WHO. Suitable methods to determine the international units of an interferon preparation are described, for example, in Familletti, P.C., et al. (Methods in Enzymol., 1981, 78; S. Pestka, ed., Academic Press, New York, pages 387-394), as well as in Finter, N.B. (J. Gen. Virol., v. 5, 419 (1969), and Brennan, G.L., and Kronenberg, L.H., (Bio.Techniques., 1, 78 (1983)).

The dose is dependent on the severity of, and the response to, the condition to be treated and may vary between several days and several months, or until the condition is observed to subside. The optimal dosage may be determined by making periodical measurements of the agent concentrations in the patient's body. The optimal dose may be determined from the effective concentration 50 (EC50) values obtained by means of previous *in vitro* or *in vivo* assays in animal models. The unit dose may be administered once a day or less than once a day, preferably, less than once every 2, 4, 8 or 30 days. Alternatively, it is possible to administer an initial dose followed by one or several maintenance doses, generally in a lower quantity than the initial dose. The maintenance regimen may involve treating the patient with doses that range between 0.01 µg and 1.4 mg/kg of body weight per day, for example, 10, 1, 0.1, 0.01, 0.001 or 0.00001 mg per kg of body weight per day. The maintenance doses are preferably administered, at most, once every 5, 10 or 30 days. The treatment must be continued for a period of time that will vary depending on the type of alteration suffered by the patient, its severity and the patient's condition. Following the treatment, the evolution of the patient must be monitored in order to determine whether the dose must be increased, in the event that the disease does not respond to the treatment, or needs to be reduced, in the event that an improvement of the disease or undesireable secondary effects are observed.

The daily dose may be administered in a single dose or in two or more doses, depending on the particular circumstances. If repeated administration or frequent administrations are desired, it is adviseable to implant an administration device, such as a pump, a semi-permanent catheter (intravenous, intraperitoneal, intracisternal or intracapsular) or a reservoir.

The components that form the compositions of the invention may be administered together as separate pharmaceutical formulations or as part of the same unitary dosage form. Alternatively, the different components of the compositions of the invention may be administered separately, but as part of the same therapeutic regimen. In the case of separate administration, the components need not be administered at essentially the same time, although they can if so desired. Thus, the OSMR agonist and the type III interferon or the functionally equivalent variant thereof may be administered simultaneously, but by different administration routes. Optionally, the separate administration of the OSMR agonist and the type III interferon or the functionally equivalent variant thereof may be performed at different times and in any order.

Thus, the invention provided a product comprising an OSMR agonist and a type III IFN or a functionally equivalent variant thereof as a combined preparation for simultaneous, separate or sequential use for the treatment of infectious or neoplastic diseases.

As such, this invention further relates to a combination of separate pharmaceutical formulations in the for of a kit. For example, a kit may comprise two separate pharmaceutical formulations comprising: 1) an OSMR agonist, and 2) a type III IFN or a functionally equivalent variant thereof The kit also comprises a container for the separate formulations, such as a divided bottle or a divided foil pocket. Some additional examples of containers include syringes, boxes, bags and the like. Normally, a kit comprises directions for the administration of the separate components. The kit form is particularly advantageous when the separate components are preferably administered in different dosage forms (for example, oral and parenteral), are administered at different dosage intervals or when titration of the individual components of the combination is desired by the prescribing physician.

The product or kit of this invention is particularly suited for enhancing the immunostimulating activity of hepatic cells in a human. In one embodiment of the present invention, the product or kit comprising an OSMR agonist and a type III interferon or a functionally equivalent variant thereof is used in the immunotherapy of cancer and diseases caused by infectious pathogens, like viruses, bacteria, fungi and parasites.

It is especially advantageous to formulate the aforementioned pharmaceutical compositions in unit dosage form for ease of administration and uniformity of dosage. Unit dosage form as used herein refers to physically discrete units suitable as unitary dosages, each unit containing a predetermined quantity of active ingredient calculated to produce the desired therapeutic effect in association with the required pharmaceutical carrier. Examples of such unit dosage forms are tablets (including scored or coated tablets), capsules, pills, suppositories, powder packets, wafers, injectable solutions or suspensions and the like, and segregated multiples thereof.

The daily dosage of the compounds according to the invention shall vary with the mode of administration, the treatment desired, and the severity of the disorder.

In general, it is contemplated that an effective daily amount of OSM may range from about 0.05 to about 5.0 mg per patient. In the case of type III IFN, the effective daily amount would be from 0.5 to 100 µg/kg, preferably doses of 0.5, 1.5, 5, 10, 20 or 40 µg/kg. The administration may be performed on a weekly basis or every two weeks. The composition may be administered subcutaneously.

In some embodiments, the type III interferon or the functionally equivalent variant thereof may be administered by means of a single administration pattern or by combining a first and a second administration pattern followed by a second administration. Said first administration (known as "induction regimen") involves the administration of a higher dose of interferon and may be performed by means of a single administration or by means of several doses administered daily, every two days, three times a week, every two weeks, 3 times a month or once a month. Said first administration is performed for a period of time that may range about at least 4 weeks, at least 8 weeks or at least 12 weeks. Said first administration may be performed simultaneously with the administration of the OSMR agonist or separately in time, and may be performed by the same administration route or a different administration route than the OSMR agonist.

The second administration regimen of the type III interferon or the functionally equivalent variant thereof (maintenance regimen) generally involves the administration of a lower quantity of said compounds. Thus, it is possible to administer at least about 3 µg, at least about 9 µg, at least about 15 µg, at least about 18 µg. The second administration regimen may include one or several daily administrations, every two days, three times a week, every two weeks, 3 times a month or once a month.

It may be appropriate to administer the required dose as one, two, three, four or more sub-doses at appropriate intervals throughout the day. Furthermore, it is evident that said effective daily amount may be lowered or increased depending on the response of the treated subject and/or depending on the evaluation of the physician prescribing the compounds of the instant invention. The effective daily amount ranges mentioned hereinabove are therefore only guidelines.

The present invention is illustrated by the following examples, which are merely intended for illustrative purposes and in no case should be considered to limit the scope of the invention.

### EXAMPLES

The following examples include comparative assays performed with compositions that comprised different combinations of the following compounds:
Human recombinant oncostatin M (OSM) obtained from R&D Systems (Minneapolis, MN, USA; Catalogue No. 295-OM; SEQ. ID. NO.: 2);

A human recombinant IFN-λ1 obtained from PeproTech (PeproTech Inc., Rocky Hill, NJ, USA; Catalogue No. 300-02L), the amino acid sequence whereof is (SEQ. ID. NO.: 6):

PTSKPTTTGK GCHIGRFKSL SPQELASFKK ARDALEESLK LKNWSCSSPV FPGNWDLRLL
QVRERPVALE AELALTLKVL EAAAGPALED VLDQPLHTLH HILSQLQACI QPQPTAGPRP
RGRLHHWLHR LQEAPKKESA GCLEASVTFN LFRLLTRDLK YVADGNLCLR TSTHPEST

A human recombinant IFN-λ2, also obtained from Peprotech (PeproTech Inc., Rocky Hill, NJ, USA; Catalogue No. 300-02K), the amino acid sequence whereof is (SEQ. ID. NO.: 10):

PVARLHGALP DARGCHIAQF KSLSPQELQA FKRAKDALEE SLLLKDCRCH SRLFPRTWDL
RQLQVRERPM ALEAELALTL KVLEATADTD PALVDVLDQP LHTLHHILSQ FRACIQPQPT
AGPRTRGRLH HWLYRLQEAP KKESPGCLEA SVTFNLFRLL TRDLNCVASG DLCV

### Example 1. Antiviral effect in Huh7 cells that express the HCV replicon. Analysis of the expression of viral mRNA.

The *in vitro* HCV replication system on the Huh7 hepatic cell line, called HCV replicon, is an experimental model that has been widely used in the literature to assess the effect of different molecules on HCV replication [Manns et al. Nat Rev Drug Discov, 2007; 6: 991-1000]. This cell line has surface receptors for the molecules assayed, IFN-λ and OSM; therefore, this system is an ideal assay to evaluate the antiviral efficacy of both molecules.

In the first place, Huh7 cells were established which expressed the full-length HCV replicon, as described by Pietschmann et al. [J Virol 2002; 76: 4008-4021]. In sum, pI389/Core-3'/5.1 were linearised with ScaI (New England Biolabs, USA) and used as templates for the synthesis of RNA using T7 RNA polymerase (Promega, USA). 20 µg of synthesised RNA were used to electroporate 10⁷ Huh7 cells and, after 24 hours, 500 µg/ml of G418 (Gibco, USA) were added. Twice a week, the culture medium supplemented with G418 was replaced and, 4 weeks after the transfection, the mixed colonies resistant to G418 were collected and used for the subsequent analysis thereof.

In order to analyse the HCV mRNA by means of quantitative real-time PCR (RT-PCR), Huh7 cells that expressed the full-length HCV replicon were seeded at 10,000/well in 96-well plates in D-MEM with 10% FCS. The corresponding substance to be assayed was added and the cell culture was maintained for three days.

The total RNA of Huh7 cells was obtained by means of automatic extraction. The total RNA was treated with DNAase (Gibco-BRL) prior to reverse transcription with M-MLV Reverse Transcriptase (Gibco-BRL) in the presence of RNaseOUT (Gibco-BRL). The expression of the mRNA of HCV and the mRNA of β-actin was measured by quantitative real-time PCR using an ICycler and the IQ SYBR Green Supermix (Bio-Rad Laboratories). 2-µl aliquots from the cDNA pool were used for each PCR, which contained specific sense and anti-sense primers for the untranslated 5'-region of HCV
5' CCTGTGAGGAACTACTGTCT 3' (SEQ. ID. NO.: 13), and
5' CTATCAGGCAGTACCACAAG 3' (SEQ. ID. NO.: 14); or, for the β-actin gene
5' AGCCTCGCCTTTGCCGA 3' (SEQ. ID. NO.: 15), and
5' CTGGTGCCTGGGGCG 3' (SEQ. ID. NO.: 16),
in a final volume of 20 µl.

In order to determine the specificity of the PCR products, their dissociation temperature was measured. The results were normalised on the basis of the quantification of β-actin in the same sample. The quantity of HCV mRNA was measured by means of the formula 2^{ct(actin)-ct(HCV)}, ct being the point at which the fluorescence appreciably increases above the background fluorescence.

The analysis of the type of interaction between IFN-λ and OSM was performed by means of multi-variant analysis in accordance with the method described by Chou [Synergism and Antagonism in chemotherapy 1991; 61-102].

The type of interaction between two substances is measured by means of a factor called interaction index "I", where I = d1/D1 + d2/D2; where d1 and d2 are the concentrations of the inhibitors in the combination, and D1 and D2 are the concentrations of inhibitors 1 and 2 that separately exert the same inhibition as the combination. Thus, if "I" is equal to 1, the substances do not react with one another (additive effect), if "I" is less than 1, the combination is synergic, and if "I" is greater than 1, the combination is antagonistic.

In order to obtain the pertinent comparisons, parallel assays were performed using the following substances to be assayed:
- Control, wherein no compounds were added;
- IFN-λ1 (50 IU/ml);
- IFN-λ2 (50 IU/ml);
- Combination of IFN-λ1 (50 IU/ml) with OSM (20 ng/ml);
- Combination of IFN-λ1 (50 IU/ml) with OSM (10 ng/ml);
- Combination of IFN-λ2 (50 IU/ml) with OSM (20 ng/ml); and
- Combination of IFN-λ2 (50 IU/ml) with OSM (10 ng/ml).

**Table 4. Study of the type of interaction established between IFN-lambda (IFN-λ1 or IFN-λ2) and OSM on the replication of HCV in Huh7 cells transfected with the full-length HCV replicon.**

| **Huh7 clone** | **Combination** | **I** |
|---|---|---|
| A | IFN-λ1 (50 IU / ml) + OSM (20 ng/ml) | 0.29 |
| | IFN-λ1 (50 IU / ml) + OSM (10 ng/ml) | 0.40 |
| | IFN-λ2 (50 IU / ml) + OSM (20 ng/ml) | 0.15 |
| | IFN-λ2 (50 IU / ml) + OSM (10 ng/ml) | 0.22 |
| B | IFN-λ1 (50 IU / ml) + OSM (10 ng/ml) | 0.38 |
| | IFN-λ2 (50 IU / ml) + OSM (10 ng/ml | 0.43 |

The letters A and B refer to the study performed on two different clones of Huh7 cells carrying the HCV genomic replicon, shown in Figure 1.

In conclusion, the experiments performed demonstrated that the combined treatment of IFN-lambda jointly with OSM causes a greater inhibition of the replication of HCV than that induced by the cytokines administered individually (Figure 1). Moreover, the analysis of the type of synergism established between both molecules demonstrated that the interaction between them is of the synergic type.

### Example 2. Antiviral effect in Huh7 cells that express the HCV replicon. Analysis of the expression of the viral Core protein.

In the first place, Huh7 cell lines carrying the full-length HCV replicon were established in the same manner as described in example 1.

Subsequently, Huh7 cells that expressed the full-length HCV replicon were seeded at 200,000/well in 6-well plates in D-MEM (Gibco) with 10% FCS (Gibco). The corresponding substance to be assayed was added and incubated for 72 hours. Thereafter, the Huh7 cells were lysed in lysis buffer (60 mM Tris-HCl, pH 6.8, 2% SDS, 2.5% glycerol, 0.7 M β-mercaptoethanol and 0.02% bromophenol blue). The samples were resolved in 15% SDS-polyacrylamide gels (Bio-Rad Laboratories, CA) under reducing conditions. Following the electrophoresis, they were transferred to nitrocellulose membranes (Bio-Rad Laboratories) and stained with Ponceau red solution (Sigma-Aldrich, Germany), in order to verify that there was an equal load of proteins. The membranes were incubated in TBS-T (50 mM Tris-HCl (pH 7.6), 200 mM NaCl and 0.1% Tween-20) with 5% dehydrated milk. The proteins were detected by incubation with the specific primary antibody (anti-Core at a 1/1,000 dilution) in TBS-T for 1 hour. Subsequently, the membranes were washed in TBS-T and peroxidase-conjugated secondary antibody was added for 1 hour. The membranes were subjected to extensive washings in TBS-T and the specific protein bands were visualised using the "Western Lightning chemiluminescence reagent plus" chemoluminescence detection system (Perkin Elmer, USA), following the manufacturer's instructions.

In order to obtain the pertinent comparisons, parallel assays were performed using the following substances to be assayed:
- Control, wherein no compounds were added;
- IFN-λ1 (50 IU/ml);
- OSM (10 ng/ml); and
- Combination of IFN-λ1 (50 IU/ml) with OSM (10 ng/ml).

As shown in Figure 2, the Western Blot analysis of the Core structural protein demonstrated a greater inhibition of viral replication when an IFN-λ is combined with OSM, confirming the results obtained in the real-time PCR analysis (Figure 1).

### Example 3. Immunomodulatory effect. Analysis of the expression of genes involved in antigen processing and presentation.

A crucial aspect in the defense against viral infections is the ability of the infected cell to present viral peptides in the cell membrane within the context of type I HLA molecules. For the viral proteins to be presented, they must be previously processed in the form of small peptides and transported to the plasma membrane. PSMB9 is one of the subunits that make up immunoproteasome, which plays a crucial role in the processing and presentation of specific peptides for cytotoxic lymphocytes [Strehl et al. Immunol Rev. 2005; 207: 19-30]. TAP 1 and TAP 2 are two critical proteins for the loading of antigenic peptides in class I HLA [Van den Eynde et al. Curr Opin Immunol., 2001; 13:147-153].

In the same manner as described in example 1, Huh7 cell lines carrying the full-length HCV replicon were established.

In order to analyse the mRNA of immunomodulatory genes by quantitative real-time PCR, Huh7 cells that expressed the full-length HCV replicon were seeded at 10,000/well in 96-well plates in D-MEM with 10% FCS. The corresponding substance to be assayed was added, the cells were collected following different incubation times (24 and 48 hours) and the expression profile of the genes involved in the cell response to an infection was analysed. The total RNA was obtained by means of automatic extraction. The total RNA was treated with DNAase (Gibco-BRL) prior to reverse transcription with M-MLV Reverse Transcriptase (Gibco-BRL) in the presence of RNaseOUT (Gibco-BRL). The expression of the mRNA of TAP 1, TAP 2 and PSMB9, and the mRNA of β-actin was measured by quantitative real-time PCR using an ICycler and the IQ SYBR Green Supermix (Bio-Rad Laboratories). 2-µl aliquots from the cDNA pool were used for each PCR, which contained specific sense and anti-sense primers for TAP1, TAP2, PSMB9 and the β-actin gene in a final volume of 20 µl. The following primers were used:
for TAP 1
5' TCGTTGTCAGTTATGCAGCG 3' (SEQ. ID. NO.: 17), and
5' CCCGTAAAGAATGGAATGGC 3' (SEQ. ID. NO.: 18);
for TAP2
5' GGACCAGGTGAACAACAAAG 3' (SEQ. ID. NO.: 19), and
5' CAAAGGAGAAGAGGCACATG 3' (SEQ. ID. NO.: 20);
for PSMB9
5' CATCATGGCAGTGGAGTTTG 3' (SEQ. ID. NO.: 21), and
5' TGAGATGTGCAGACAAGTCC 3' (SEQ. ID. NO.: 22); and
for β-actin
5' AGCCTCGCCTTTGCCGA 3' (SEQ. ID. NO.: 15), and
5' CTGGTGCCTGGGGCG 3' (SEQ. ID. NO.: 16).

In order to obtain the pertinent comparisons, parallel assays were performed using the following substances to be assayed:
- Control, wherein no compounds were added;
- IFN-λ1 (50 IU/ml);
- IFN-λ2 (50 IU/ml);
- OSM (10 ng/ml);
- Combination of IFN-λ1 (50 IU/ml) with OSM (10 ng/ml); and
- Combination of IFN-λ2 (50 IU/ml) with OSM (10 ng/ml).

As shown in Figure 3, the results obtained demonstrated a higher expression of genes involved in antigen processing and presentation, such as TAP1, TAP2 and PSMB9, when an IFN-λ is combined with OSM in Huh7 cells carrying the HCV genomic replicon. These results show that the combined action of IFN-lambda and OSM enhances immunostimulating properties in the hepatic cell. This genetic profile is a key to the resolution of processes that alter cellular homeostasis, such as viral infections or the acquisition of a tumoural phenotype.

### Example 4. Effect of the combination of IFN-λ1 with OSM on the signalling cascade in hepatoma cells -Huh- or in cells that maintain HCV replication.

Several components of the Jak-STAT signalling cascade (STAT1, STAT2, STAT3 and STAT5) were analysed, following treatment with cytokines IFN-λ1 and/or OSM, which activate this signalling pathway.

Figure 4 shows the quantity of protein activated (phosphorylated) and the total quantity of protein present in human hepatoma cells (Huh7) in the presence (Huh7-Core 3') or absence (Huh7) of the HCV genomic replicon at different times (3, 12, 24 and 48 hours) following the addition of the substance to be assayed. After the specified times had elapsed, cell extracts were prepared wherein the quantity of phosphorylated and total STAT1, STAT2, STAT3 and STAT5 present was examined by means of SDS-PAGE/Western-blot. An analysis of the quantity of actin present in each of the samples was included, which showed that in all cases the same quantity of cell extract had been loaded.

The antibodies used for the analysis were: anti-phospho-STAT-1^{Tyr701} (9171L/Cell signaling technology), anti-phospho-STAT-2 (07-224/Upstate), anti-phospho-STAT-3^{Tyr705} (9131/ Cell signaling technology), anti-phospho-STAT-5 (9351S/ Cell signaling technology), anti-STAT-1 (sc-346), anti-STAT-2 (06-502/Upsate), anti-STAT-3 (06596/Upstate), anti-STAT-5 (9363/Cell signaling technology) and anti-actin (A2066/Sigma Aldrich).

Parallel assays were performed using the following substances to be assayed:
- Control, wherein no compounds were added,
- IFN-λ1 (5,000 IU/ml),
- Oncostatin M (OSM, 10 ng/ml), and
- the combination of IFN-λ1 (5,000 IU/ml) with OSM (10 ng/ml)

The incubation of Huh7 cells and Huh7-Core-3' cells, with IFN-λ, OSM or the combination of IFN-λ1 and OSM induces the phosphorylation of STAT1, STAT2, STAT3 and STAT5, the activation of STAT2 being specific for IFN-λ1 and that of STAT3 being specific for OSM. Upon studying the results of the combined use of both cytokines, a continuous activation in time of the four proteins analysed is observed, and equal (STAT3 and STAT5) or higher (STAT1 and STAT2) activation levels are achieved than with the cytokines used in isolation.

The isolated administration of IFN-λ1 generates an increase in time of the total quantity of STAT1 present in the cells and OSM of the total quantity of STAT3. These effects are reproduced when both cytokines are administered jointly, and an increase in the total quantity of STAT2 detected in the cells is additionally observed.

Following these experiments, it may be concluded that the combined use of both cytokines is capable of reproducing the effects induced by each of the cytokines (activation of STAT3 and STAT5) in isolation, but, moreover, causes a significant increase in the quantity of STAT1 and STAT2 activated. Thus, a larger number of active transcriptional complexes may be generated and the biological activity may be increased by the combined use of both cytokines as compared to their individual use. The enhancement of the activation of STAT2 is particularly relevant, since, unlike STAT1, STAT3 and STAT5, this molecule is only activated by IFN-λ1.

## Claims

1. Composition or kit comprising, together or separately,
(i) a first component selected from the group of
(a) an OSMR agonist and
(b) a polynucleotide that encodes an OSMR agonist and
(ii) a second component selected from the group of
(a) at least one type III interferon or a functionally equivalent variant thereof, and
(b) at least one polynucleotide that encodes a type III interferon or a functionally equivalent variant thereof.

2. Composition or kit according to claim 1, wherein the OSMR agonist is an oncostatin M.

3. Composition or kit according to claim 2, wherein the oncostatin M comprises the amino acid sequence identified as SEQ. ID. NO.:2.

4. Composition or kit according to any of claims 1 to 3, wherein the type III interferon is selected from the group consisting of an interferon-λ1, an interferon-λ2, an interferon-λ3 and a combination of one or more thereof

5. Composition or kit according to claim 4, wherein the interferon-λ1 comprises an amino acid sequence selected from SEQ. ID. NO.:4, SEQ. ID. NO.:5, SEQ. ID. NO.:6 and SEQ. ID. NO.:7, the interferon-λ2 comprises a sequence selected from SEQ. ID. NO.:9 and SEQ. ID. NO.:10, and/or the interferon-λ3 comprises sequence SEQ. ID. Neo.:12.

6. Composition or kit according to claims 5 or 6, wherein the type III interferon is human pegylated interferon-λ1.

7. A pharmaceutical composition comprising a composition or kit according to any of claims 1 to 6, and at least one pharmaceutically acceptable carrier.

8. A composition or kit according to any of claims 1 to 6, or a pharmaceutical composition according to claim 7, for use in medicine.

9. Use of a composition or kit according to any of claims 1 to 6, or a pharmaceutical composition according to claim 7, for the manufacture of a medicament for the treatment of an infectious disease and/or a neoplastic disease.

10. Use according to claim 9, wherein the infectious and/or neoplastic disease is a hepatic disease.

11. Use according to claims 9 or 10, wherein the infectious disease is of viral origin.

12. Use according to claim 11, wherein the infectious disease of viral origin is a viral hepatitis.

13. Use according to claim 12, wherein the viral hepatitis is hepatitis C.

14. Use according to claims 9 or 10, wherein the neoplastic disease is an epithelial cancer.

15. Use according to claim 14, wherein the epithelial cancer is a melanoma.


SEQUENCE LISTING

<110>  PROYECTO DE BIOMEDICINA CIMA S.L.
       DIGNA BIOTECH S.L.

<120>  COMPOSITIONS FOR THE TREATMENT OF INFECTIOUS AND TUMOURAL
       DISEASES

<130>  P5554PC00

<150>  P201030170
<151>  2010-02-09

<160>  22

<170>  PatentIn version 3.5

<210>  1
<211>  252
<212>  PRT
<213>  Homo sapiens


<220>
<221>  MISC_FEATURE
<223>  Human Oncostatin M (OSM) precursor (Uniprot Accesion No. P13725)

<400>  1

Met Gly Val Leu Leu Thr Gln Arg Thr Leu Leu Ser Leu Val Leu Ala
1               5                   10                  15


Leu Leu Phe Pro Ser Met Ala Ser Met Ala Ala Ile Gly Ser Cys Ser
            20                  25                  30


Lys Glu Tyr Arg Val Leu Leu Gly Gln Leu Gln Lys Gln Thr Asp Leu
        35                  40                  45


Met Gln Asp Thr Ser Arg Leu Leu Asp Pro Tyr Ile Arg Ile Gln Gly
    50                  55                  60


Leu Asp Val Pro Lys Leu Arg Glu His Cys Arg Glu Arg Pro Gly Ala
65                  70                  75                  80


Phe Pro Ser Glu Glu Thr Leu Arg Gly Leu Gly Arg Arg Gly Phe Leu
                85                  90                  95


Gln Thr Leu Asn Ala Thr Leu Gly Cys Val Leu His Arg Leu Ala Asp
            100                 105                 110


Leu Glu Gln Arg Leu Pro Lys Ala Gln Asp Leu Glu Arg Ser Gly Leu
        115                 120                 125


Asn Ile Glu Asp Leu Glu Lys Leu Gln Met Ala Arg Pro Asn Ile Leu
    130                 135                 140

Gly Leu Arg Asn Asn Ile Tyr Cys Met Ala Gln Leu Leu Asp Asn Ser
145                 150                 155                 160

Asp Thr Ala Glu Pro Thr Lys Ala Gly Arg Gly Ala Ser Gln Pro Pro
                165                 170                 175

Thr Pro Thr Pro Ala Ser Asp Ala Phe Gln Arg Lys Leu Glu Gly Cys
            180                 185                 190

Arg Phe Leu His Gly Tyr His Arg Phe Met His Ser Val Gly Arg Val
        195                 200                 205

Phe Ser Lys Trp Gly Glu Ser Pro Asn Arg Ser Arg Arg His Ser Pro
    210                 215                 220

His Gln Ala Leu Arg Lys Gly Val Arg Arg Thr Arg Pro Ser Arg Lys
225                 230                 235                 240

Gly Lys Arg Leu Met Thr Arg Gly Gln Leu Pro Arg
                245                 250

<210>  2
<211>  196
<212>  PRT
<213>  Homo sapiens

<220>
<221>  MISC_FEATURE
<223>  Oncostatin M (P13725|26-221)

<400>  2

Ala Ala Ile Gly Ser Cys Ser Lys Glu Tyr Arg Val Leu Leu Gly Gln
1               5                   10                  15

Leu Gln Lys Gln Thr Asp Leu Met Gln Asp Thr Ser Arg Leu Leu Asp
            20                  25                  30

Pro Tyr Ile Arg Ile Gln Gly Leu Asp Val Pro Lys Leu Arg Glu His
        35                  40                  45

Cys Arg Glu Arg Pro Gly Ala Phe Pro Ser Glu Glu Thr Leu Arg Gly
    50                  55                  60

Leu Gly Arg Arg Gly Phe Leu Gln Thr Leu Asn Ala Thr Leu Gly Cys
65                  70                  75                  80

Val Leu His Arg Leu Ala Asp Leu Glu Gln Arg Leu Pro Lys Ala Gln
                85                  90                  95

Asp Leu Glu Arg Ser Gly Leu Asn Ile Glu Asp Leu Glu Lys Leu Gln

100                 105                 110

Met Ala Arg Pro Asn Ile Leu Gly Leu Arg Asn Asn Ile Tyr Cys Met
        115                 120                 125

Ala Gln Leu Leu Asp Asn Ser Asp Thr Ala Glu Pro Thr Lys Ala Gly
    130                 135                 140

Arg Gly Ala Ser Gln Pro Pro Thr Pro Thr Pro Ala Ser Asp Ala Phe
145                 150                 155                 160

Gln Arg Lys Leu Glu Gly Cys Arg Phe Leu His Gly Tyr His Arg Phe
                165                 170                 175

Met His Ser Val Gly Arg Val Phe Ser Lys Trp Gly Glu Ser Pro Asn
            180                 185                 190

Arg Ser Arg Arg
        195

<210>  3
<211>  200
<212>  PRT
<213>  Homo sapiens

<220>
<221>  MISC_FEATURE
<223>  Human Interleukin 29 precursor (IL29; interferon-lambda 1)
       (NP_742152.1; Uniprot Accesion No. Q8IU54)

<400>  3

Met Ala Ala Ala Trp Thr Val Val Leu Val Thr Leu Val Leu Gly Leu
1               5                   10                  15

Ala Val Ala Gly Pro Val Pro Thr Ser Lys Pro Thr Thr Thr Gly Lys
            20                  25                  30

Gly Cys His Ile Gly Arg Phe Lys Ser Leu Ser Pro Gln Glu Leu Ala
        35                  40                  45

Ser Phe Lys Lys Ala Arg Asp Ala Leu Glu Glu Ser Leu Lys Leu Lys
    50                  55                  60

Asn Trp Ser Cys Ser Ser Pro Val Phe Pro Gly Asn Trp Asp Leu Arg
65                  70                  75                  80

Leu Leu Gln Val Arg Glu Arg Pro Val Ala Leu Glu Ala Glu Leu Ala
                85                  90                  95

Leu Thr Leu Lys Val Leu Glu Ala Ala Ala Gly Pro Ala Leu Glu Asp

100                 105                 110

Val Leu Asp Gln Pro Leu His Thr Leu His His Ile Leu Ser Gln Leu
        115                 120                 125

Gln Ala Cys Ile Gln Pro Gln Pro Thr Ala Gly Pro Arg Pro Arg Gly
    130                 135                 140

Arg Leu His His Trp Leu His Arg Leu Gln Glu Ala Pro Lys Lys Glu
145                 150                 155                 160

Ser Ala Gly Cys Leu Glu Ala Ser Val Thr Phe Asn Leu Phe Arg Leu
                165                 170                 175

Leu Thr Arg Asp Leu Lys Tyr Val Ala Asp Gly Asn Leu Cys Leu Arg
            180                 185                 190

Thr Ser Thr His Pro Glu Ser Thr
        195                 200

<210>  4
<211>  181
<212>  PRT
<213>  Homo sapiens

<220>
<221>  MISC_FEATURE
<223>  Human mature IL29 (IL29; interferon-lambda 1; (Q8IU54|20-200)

<400>  4

Gly Pro Val Pro Thr Ser Lys Pro Thr Thr Thr Gly Lys Gly Cys His
1               5                   10                  15

Ile Gly Arg Phe Lys Ser Leu Ser Pro Gln Glu Leu Ala Ser Phe Lys
            20                  25                  30

Lys Ala Arg Asp Ala Leu Glu Glu Ser Leu Lys Leu Lys Asn Trp Ser
        35                  40                  45

Cys Ser Ser Pro Val Phe Pro Gly Asn Trp Asp Leu Arg Leu Leu Gln
    50                  55                  60

Val Arg Glu Arg Pro Val Ala Leu Glu Ala Glu Leu Ala Leu Thr Leu
65                  70                  75                  80

Lys Val Leu Glu Ala Ala Ala Gly Pro Ala Leu Glu Asp Val Leu Asp
                85                  90                  95

Gln Pro Leu His Thr Leu His His Ile Leu Ser Gln Leu Gln Ala Cys
            100                 105                 110

Ile Gln Pro Gln Pro Thr Ala Gly Pro Arg Pro Arg Gly Arg Leu His
        115                 120                 125

His Trp Leu His Arg Leu Gln Glu Ala Pro Lys Lys Glu Ser Ala Gly
    130                 135                 140

Cys Leu Glu Ala Ser Val Thr Phe Asn Leu Phe Arg Leu Leu Thr Arg
145                 150                 155                 160

Asp Leu Lys Tyr Val Ala Asp Gly Asn Leu Cys Leu Arg Thr Ser Thr
                165                 170                 175

His Pro Glu Ser Thr
            180

<210>  5
<211>  176
<212>  PRT
<213>  Artificial Sequence

<220>
<223>  PEG-rhIL-29 (Zymogenetics) polypeptide sequence

<400>  5

Met Lys Pro Thr Thr Thr Gly Lys Gly Cys His Ile Gly Arg Phe Lys
1               5                   10                  15

Ser Leu Ser Pro Gln Glu Leu Ala Ser Phe Lys Lys Ala Arg Asp Ala
            20                  25                  30

Leu Glu Glu Ser Leu Lys Leu Lys Asn Trp Ser Cys Ser Ser Pro Val
        35                  40                  45

Phe Pro Gly Asn Trp Asp Leu Arg Leu Leu Gln Val Arg Glu Arg Pro
    50                  55                  60

Val Ala Leu Glu Ala Glu Leu Ala Leu Thr Leu Lys Val Leu Glu Ala
65                  70                  75                  80

Ala Ala Gly Pro Ala Leu Glu Asp Val Leu Asp Gln Pro Leu His Thr
                85                  90                  95

Leu His His Ile Leu Ser Gln Leu Gln Ala Cys Ile Gln Pro Gln Pro
            100                 105                 110

Thr Ala Gly Pro Arg Pro Arg Gly Arg Leu His His Trp Leu His Arg
        115                 120                 125

Leu Gln Glu Ala Pro Lys Lys Glu Ser Ala Gly Cys Leu Glu Ala Ser

130                 135                 140

Val Thr Phe Asn Leu Phe Arg Leu Leu Thr Arg Asp Leu Lys Tyr Val
145                 150                 155                 160

Ala Asp Gly Asn Leu Ser Leu Arg Thr Ser Thr His Pro Glu Ser Thr
                165                 170                 175

<210>  6
<211>  178
<212>  PRT
<213>  Artificial Sequence

<220>
<223>  Recombinant human IL29 (Preprotech Code N. 300-02L)

<220>
<221>  MISC_FEATURE
<223>  Recombinant Human IFN-lambda 1 (IL-29) (PeproTech Code
       No.300-02L)

<400>  6

Pro Thr Ser Lys Pro Thr Thr Thr Gly Lys Gly Cys His Ile Gly Arg
1               5                   10                  15

Phe Lys Ser Leu Ser Pro Gln Glu Leu Ala Ser Phe Lys Lys Ala Arg
            20                  25                  30

Asp Ala Leu Glu Glu Ser Leu Lys Leu Lys Asn Trp Ser Cys Ser Ser
        35                  40                  45

Pro Val Phe Pro Gly Asn Trp Asp Leu Arg Leu Leu Gln Val Arg Glu
    50                  55                  60

Arg Pro Val Ala Leu Glu Ala Glu Leu Ala Leu Thr Leu Lys Val Leu
65                  70                  75                  80

Glu Ala Ala Ala Gly Pro Ala Leu Glu Asp Val Leu Asp Gln Pro Leu
                85                  90                  95

His Thr Leu His His Ile Leu Ser Gln Leu Gln Ala Cys Ile Gln Pro
            100                 105                 110

Gln Pro Thr Ala Gly Pro Arg Pro Arg Gly Arg Leu His His Trp Leu
        115                 120                 125

His Arg Leu Gln Glu Ala Pro Lys Lys Glu Ser Ala Gly Cys Leu Glu
    130                 135                 140

Ala Ser Val Thr Phe Asn Leu Phe Arg Leu Leu Thr Arg Asp Leu Lys
145                 150                 155                 160

Tyr Val Ala Asp Gly Asn Leu Cys Leu Arg Thr Ser Thr His Pro Glu
                165                 170                 175

Ser Thr

<210>  7
<211>  176
<212>  PRT
<213>  Homo sapiens

<400>  7

Met Lys Pro Thr Thr Thr Gly Lys Gly Cys His Ile Gly Arg Phe Lys
1               5                   10                  15

Ser Leu Ser Pro Gln Glu Leu Ala Ser Phe Lys Lys Ala Arg Asp Ala
            20                  25                  30

Leu Glu Glu Ser Leu Lys Leu Lys Asn Trp Ser Cys Ser Ser Pro Val
        35                  40                  45

Phe Pro Gly Asn Trp Asp Leu Arg Leu Leu Gln Val Arg Glu Arg Pro
    50                  55                  60

Val Ala Leu Glu Ala Glu Leu Ala Leu Thr Leu Lys Val Leu Glu Ala
65                  70                  75                  80

Ala Ala Gly Pro Ala Leu Glu Asp Val Leu Asp Gln Pro Leu His Thr
                85                  90                  95

Leu His His Ile Leu Ser Gln Leu Gln Ala Cys Ile Gln Pro Gln Pro
            100                 105                 110

Thr Ala Gly Pro Arg Pro Arg Gly Arg Leu His His Trp Leu His Arg
        115                 120                 125

Leu Gln Glu Ala Pro Lys Lys Glu Ser Ala Gly Cys Leu Glu Ala Ser
    130                 135                 140

Val Thr Phe Asn Leu Phe Arg Leu Leu Thr Arg Asp Leu Lys Tyr Val
145                 150                 155                 160

Ala Asp Gly Asn Leu Cys Leu Arg Thr Ser Thr His Pro Glu Ser Thr
                165                 170                 175

<210>  8
<211>  200
<212>  PRT
<213>  Homo sapiens

<220>
<221>  MISC_FEATURE
<223>  Interleukin 28A precursor (IL28A; interferon-lambda 2)
       (NP_742150.1; Uniprot Accesion No. Q8IZJ0)

<400>  8

Met Lys Leu Asp Met Thr Gly Asp Cys Thr Pro Val Leu Val Leu Met
1               5                   10                  15

Ala Ala Val Leu Thr Val Thr Gly Ala Val Pro Val Ala Arg Leu His
            20                  25                  30

Gly Ala Leu Pro Asp Ala Arg Gly Cys His Ile Ala Gln Phe Lys Ser
        35                  40                  45

Leu Ser Pro Gln Glu Leu Gln Ala Phe Lys Arg Ala Lys Asp Ala Leu
    50                  55                  60

Glu Glu Ser Leu Leu Leu Lys Asp Cys Arg Cys His Ser Arg Leu Phe
65                  70                  75                  80

Pro Arg Thr Trp Asp Leu Arg Gln Leu Gln Val Arg Glu Arg Pro Met
                85                  90                  95

Ala Leu Glu Ala Glu Leu Ala Leu Thr Leu Lys Val Leu Glu Ala Thr
            100                 105                 110

Ala Asp Thr Asp Pro Ala Leu Val Asp Val Leu Asp Gln Pro Leu His
        115                 120                 125

Thr Leu His His Ile Leu Ser Gln Phe Arg Ala Cys Ile Gln Pro Gln
    130                 135                 140

Pro Thr Ala Gly Pro Arg Thr Arg Gly Arg Leu His His Trp Leu Tyr
145                 150                 155                 160

Arg Leu Gln Glu Ala Pro Lys Lys Glu Ser Pro Gly Cys Leu Glu Ala
                165                 170                 175

Ser Val Thr Phe Asn Leu Phe Arg Leu Leu Thr Arg Asp Leu Asn Cys
            180                 185                 190

Val Ala Ser Gly Asp Leu Cys Val
        195                 200


<210>  9
<211>  175
<212>  PRT
<213>  Homo sapiens

<220>
<221>  MISC_FEATURE
<223>  Human mature IL28A (IL28A; interferon-lambda 2; Q8IZJ0|26-200)

<400>  9

Val Pro Val Ala Arg Leu His Gly Ala Leu Pro Asp Ala Arg Gly Cys
1               5                   10                  15

His Ile Ala Gln Phe Lys Ser Leu Ser Pro Gln Glu Leu Gln Ala Phe
            20                  25                  30

Lys Arg Ala Lys Asp Ala Leu Glu Glu Ser Leu Leu Leu Lys Asp Cys
        35                  40                  45

Arg Cys His Ser Arg Leu Phe Pro Arg Thr Trp Asp Leu Arg Gln Leu
    50                  55                  60

Gln Val Arg Glu Arg Pro Met Ala Leu Glu Ala Glu Leu Ala Leu Thr
65                  70                  75                  80

Leu Lys Val Leu Glu Ala Thr Ala Asp Thr Asp Pro Ala Leu Val Asp
                85                  90                  95

Val Leu Asp Gln Pro Leu His Thr Leu His His Ile Leu Ser Gln Phe
            100                 105                 110

Arg Ala Cys Ile Gln Pro Gln Pro Thr Ala Gly Pro Arg Thr Arg Gly
        115                 120                 125

Arg Leu His His Trp Leu Tyr Arg Leu Gln Glu Ala Pro Lys Lys Glu
    130                 135                 140

Ser Pro Gly Cys Leu Glu Ala Ser Val Thr Phe Asn Leu Phe Arg Leu
145                 150                 155                 160

Leu Thr Arg Asp Leu Asn Cys Val Ala Ser Gly Asp Leu Cys Val
                165                 170                 175

<210>  10
<211>  174
<212>  PRT
<213>  Artificial Sequence

<220>
<223>  Recombinant IL-28A

<220>
<221>  MISC_FEATURE
<223>  Human recombinant IFN-lambda 2 (IL-28A) (PeproTech Code No.
       300-02K)

<400>  10

Pro Val Ala Arg Leu His Gly Ala Leu Pro Asp Ala Arg Gly Cys His
1               5                   10                  15

Ile Ala Gln Phe Lys Ser Leu Ser Pro Gln Glu Leu Gln Ala Phe Lys
            20                  25                  30

Arg Ala Lys Asp Ala Leu Glu Glu Ser Leu Leu Leu Lys Asp Cys Arg
        35                  40                  45

Cys His Ser Arg Leu Phe Pro Arg Thr Trp Asp Leu Arg Gln Leu Gln
    50                  55                  60

Val Arg Glu Arg Pro Met Ala Leu Glu Ala Glu Leu Ala Leu Thr Leu
65                  70                  75                  80

Lys Val Leu Glu Ala Thr Ala Asp Thr Asp Pro Ala Leu Val Asp Val
                85                  90                  95

Leu Asp Gln Pro Leu His Thr Leu His His Ile Leu Ser Gln Phe Arg
            100                 105                 110

Ala Cys Ile Gln Pro Gln Pro Thr Ala Gly Pro Arg Thr Arg Gly Arg
        115                 120                 125

Leu His His Trp Leu Tyr Arg Leu Gln Glu Ala Pro Lys Lys Glu Ser
    130                 135                 140

Pro Gly Cys Leu Glu Ala Ser Val Thr Phe Asn Leu Phe Arg Leu Leu
145                 150                 155                 160

Thr Arg Asp Leu Asn Cys Val Ala Ser Gly Asp Leu Cys Val
                165                 170

<210>  11
<211>  200
<212>  PRT
<213>  Homo sapiens

<220>
<221>  MISC_FEATURE
<223>  Human interleukin 28B precursor  (IL28B; interferon-lambda 3)
       (NP_742151.2; UniProt Accesion No. Q8IZI9)

<400>  11

Met Lys Leu Asp Met Thr Gly Asp Cys Met Pro Val Leu Val Leu Met
1               5                   10                  15

Ala Ala Val Leu Thr Val Thr Gly Ala Val Pro Val Ala Arg Leu Arg

20                  25                  30

Gly Ala Leu Pro Asp Ala Arg Gly Cys His Ile Ala Gln Phe Lys Ser
        35                  40                  45

Leu Ser Pro Gln Glu Leu Gln Ala Phe Lys Arg Ala Lys Asp Ala Leu
    50                  55                  60

Glu Glu Ser Leu Leu Leu Lys Asp Cys Lys Cys Arg Ser Arg Leu Phe
65                  70                  75                  80

Pro Arg Thr Trp Asp Leu Arg Gln Leu Gln Val Arg Glu Arg Pro Val
                85                  90                  95

Ala Leu Glu Ala Glu Leu Ala Leu Thr Leu Lys Val Leu Glu Ala Thr
            100                 105                 110

Ala Asp Thr Asp Pro Ala Leu Gly Asp Val Leu Asp Gln Pro Leu His
        115                 120                 125

Thr Leu His His Ile Leu Ser Gln Leu Arg Ala Cys Ile Gln Pro Gln
    130                 135                 140

Pro Thr Ala Gly Pro Arg Thr Arg Gly Arg Leu His His Trp Leu His
145                 150                 155                 160

Arg Leu Gln Glu Ala Pro Lys Lys Glu Ser Pro Gly Cys Leu Glu Ala
                165                 170                 175

Ser Val Thr Phe Asn Leu Phe Arg Leu Leu Thr Arg Asp Leu Asn Cys
            180                 185                 190

Val Ala Ser Gly Asp Leu Cys Val
        195                 200

<210>  12
<211>  175
<212>  PRT
<213>  Homo sapiens

<400>  12

Val Pro Val Ala Arg Leu Arg Gly Ala Leu Pro Asp Ala Arg Gly Cys
1               5                   10                  15

His Ile Ala Gln Phe Lys Ser Leu Ser Pro Gln Glu Leu Gln Ala Phe
            20                  25                  30

Lys Arg Ala Lys Asp Ala Leu Glu Glu Ser Leu Leu Leu Lys Asp Cys
        35                  40                  45

Lys Cys Arg Ser Arg Leu Phe Pro Arg Thr Trp Asp Leu Arg Gln Leu
    50                  55                  60

Gln Val Arg Glu Arg Pro Val Ala Leu Glu Ala Glu Leu Ala Leu Thr
65                  70                  75                  80

Leu Lys Val Leu Glu Ala Thr Ala Asp Thr Asp Pro Ala Leu Gly Asp
                85                  90                  95

Val Leu Asp Gln Pro Leu His Thr Leu His His Ile Leu Ser Gln Leu
            100                 105                 110

Arg Ala Cys Ile Gln Pro Gln Pro Thr Ala Gly Pro Arg Thr Arg Gly
        115                 120                 125

Arg Leu His His Trp Leu His Arg Leu Gln Glu Ala Pro Lys Lys Glu
    130                 135                 140

Ser Pro Gly Cys Leu Glu Ala Ser Val Thr Phe Asn Leu Phe Arg Leu
145                 150                 155                 160

Leu Thr Arg Asp Leu Asn Cys Val Ala Ser Gly Asp Leu Cys Val
                165                 170                 175

<210>  13
<211>  20
<212>  DNA
<213>  Artificial Sequence

<220>
<223>  Primer

<400>  13
cctgtgagga actactgtct                                                   20

<210>  14
<211>  20
<212>  DNA
<213>  Artificial Sequence

<220>
<223>  Primer

<400>  14
ctatcaggca gtaccacaag                                                   20

<210>  15
<211>  17
<212>  DNA
<213>  Artificial Sequence

<220>
<223>  Primer

<400>  15
agcctcgcct ttgccga                                                      17


<210>  16
<211>  15
<212>  DNA
<213>  Artificial Sequence

<220>
<223>  Primer

<400>  16
ctggtgcctg gggcg                                                        15


<210>  17
<211>  20
<212>  DNA
<213>  Artificial Sequence

<220>
<223>  Primer

<400>  17
tcgttgtcag ttatgcagcg                                                   20


<210>  18
<211>  20
<212>  DNA
<213>  Artificial Sequence

<220>
<223>  Primer

<400>  18
cccgtaaaga atggaatggc                                                   20


<210>  19
<211>  20
<212>  DNA
<213>  Artificial Sequence

<220>
<223>  Primer

<400>  19
ggaccaggtg aacaacaaag                                                   20


<210>  20
<211>  20
<212>  DNA
<213>  Artificial Sequence

<220>
<223>  Primer

<400>  20
caaaggagaa gaggcacatg                                                   20


<210>  21

<211>  20
<212>  DNA
<213>  Artificial Sequence

<220>
<223>  Primer

<400>  21
catcatggca gtggagtttg                                                    20


<210>  22
<211>  20
<212>  DNA
<213>  Artificial Sequence

<220>
<223>  Primer

<400>  22
tgagatgtgc agacaagtcc                                                    20